# EUROPEAN PATENT APPLICATION

(11) **EP 3 995 134 A1**
(43) Date of publication of application: **11.05.2022**
(21) Application number: 20836327.5
(22) Date of filing: 06.07.2020
(51) Int. Cl.: A61K 9/127, A61K 31/285, A61K 33/36, A61P 25/00

(54) **MICROMOLECULE PI4KIIIALPHA INHIBITOR COMPOSITION, PREPARATION METHOD THEREFOR AND USE THEREOF**

(30) Priority: 05.07.2019 WO PCT/CN2019/094831
(71) Applicant: Nuo-Beta Pharmaceutical Technology (Shanghai) Co., Ltd., Shanghai 201210 (CN)
(72) Inventor: HUANG, Fude, Shanghai 201199 (CN); WANG, Feng, Shanghai 201203 (CN); YANG, Shu, Shanghai 201203 (CN); JIAO, Changping, Shanghai 200120 (CN); ZHOU, Xiaojun, Shanghai 200433 (CN)
(74) Representative: Lahrtz, Fritz
(86) International application number: PCT/CN2020/100377
(87) International publication number: WO 2021/004422

(57) **Abstract**

Disclosed is a pharmaceutical composition comprising a micromolecule PI4KIIIα inhibitor and a pharmaceutically acceptable carrier, wherein the pharmaceutically acceptable carrier comprises a lipid. Also disclosed are a method for preparing the pharmaceutical composition, and a method for treating PI4KIIIα-related diseases by using the pharmaceutical composition.

## Description

### TECHNICAL FIELD

The present invention relates to a pharmaceutical composition, in particular to a pharmaceutical composition comprising a therapeutically effective amount of a micromolecule PI4KIIIα inhibitor and a pharmaceutically acceptable carrier. The present invention further relates to a preparation method for the pharmaceutical composition and use thereof.

### BACKGROUND

Phosphatidylinositol 4-kinase (PI4KIIIα) is a kinase capable of catalyzing phosphorylation of a D4 position on a phosphatidyl inositol (PI) ring to produce 4-phosphatidyl-inositide (PI4P). The PI4P is then catalyzed by PIP5-K kinases to generate 4,5-phosphatidyl-inosididediphosphate (PIP2), and the PIP2 is a direct catalytic substrate of a PI3K, can activate the activities of multiple downstream proteins and plays a key role in PI3K/Akt. Therefore, the PI4KIIIα indirectly affects a PI3K/Akt signaling pathway by affecting the PIP2, and a PI4KIIIα inhibitor can be thus used for treating diseases related to the PI3K/Akt signaling pathway.

Particularly, studies have shown that the PI4P, a product of the PI4KIIIα, is significantly increased in the cerebral cortex of an Alzheimer's disease (AD) patient, and the increased level is closely related to the degree of cognitive dysfunction in the AD patient (Zhu, L., et al., Proc Natl Acad Sci USA, 2015). In AD models of cultured cells, drosophilae and mice, inhibiting the PI4KIIIα through genetic methods or compounds can promote the release of β-amyloid peptide 42 (Aβ42) from cells and relieve neurological damage on the AD animal models, including synaptic transmission as well as learning and memory disorders (Zhang, X., et al, J. Neurosci, 2017; Zhang et al., 2017;Huang. FD., et al., PCT/CN2016/080907). Therefore, the PI4KIIIα kinase inhibitor can effectively treat the AD.

The PI4KIIIα inhibitor may have many therapeutic uses, but such inhibitor has the disadvantages such as low water solubility and poor stability. The PI4KIIIα inhibitor may be delivered by organic solvents commonly used for such medicament or other methods that promote the solubilization of such medicament in water, but the use of such preparations to deliver the PI4KIIIα inhibitor in vivo leads to poor bioavailability, it is impossible to avoid or reduce the toxicity of the medicament itself in the body (e.g., in the digestive tract), and the organic solvents themselves also have a risk of potential toxicity. Therefore, there is currently a need for a pharmaceutical preparation of the PI4KIIIα inhibitor that can be effectively delivered and minimize the toxicity of active substances.

### SUMMARY

The present disclosure provides a pharmaceutical composition comprising a micromolecule PI4KIIIα inhibitor and a pharmaceutically acceptable carrier, wherein the pharmaceutically acceptable carrier includes a lipid.

In some embodiments, the micromolecule PI4KIIIα inhibitor is PAO and a derivative of PAO.

In some embodiments, the micromolecule PI4KIIIα inhibitor has a structure of formula (I) or a pharmaceutically acceptable salt thereof, 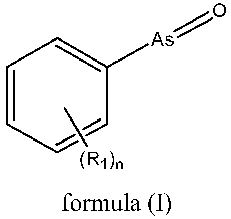 wherein R₁ is each independently selected from H, halogen, nitro, cyano, hydroxyl, amino, carbamoyl, C₁₋₆ alkyl, C₁₋₆ alkoxy, C₁₋₆ haloalkyl, -As(O), -NH-(C₁₋₆ alkyl), N,N-(C₁₋₆ alkyl)₂, -NH-C(O)-R₂, -NH-S(O)₂-R₃, -C(O)OR₄ or heterocyclyl, wherein n is an integer of 0-5, R₂ and R₃ are each independently selected from H, amino, C₁₋₆ alkyl, C₁₋₆ alkoxy, C₁₋₆ haloalkyl, -NH-(C₁₋₆ alkyl), N,N-(C₁₋₆ alkyl)₂, -C(O)OR₄, C₃₋₆ cycloalkyl, 6-12 membered aryl or 3-6 membered heterocyclyl, which are optionally substituted by halogen, nitro, cyano, hydroxyl, amino, carbamoyl, aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, and R₄ is C₁₋₆ alkyl.

In some embodiments, R₁ is each independently selected from H, halogen, nitro, cyano, hydroxyl, amino, carbamoyl, C₁₋₆ alkyl, C₁₋₆ alkoxy, C₁₋₆ haloalkyl, -As(O), - NH-(C₁₋₆ alkyl), N,N-(C₁₋₆ alkyl)₂ or -C(O)OR₄, wherein n is an integer of 0-2, and R₄ is C₁₋₆ alkyl. In some embodiments, R₁ is each independently selected from H, halogen, nitro, cyano, hydroxyl, amino, C₁₋₆ alkyl, C₁₋₆ alkoxy, C₁₋₆ haloalkyl or -As(O), wherein n is an integer of 0-2. In some embodiments, R₁ is each independently selected from H, halogen, amino or C₁₋₆ alkoxy, wherein n is 1. In some embodiments, R₁ is located at an ortho position or a para position of the -As(O) group. In some embodiments, R₁ is H.

In some embodiments, the micromolecule PI4KIIIα inhibitor is at an amount of 0.01-20 mg/g, 0.05-20 mg/g, 0.1-20 mg/g, 0.2-20 mg/g, 0.5-20 mg/g, 0.8-20 mg/g, 1-20 mg/g, 1-18 mg/g, 1-16 mg/g, 1-14 mg/g, 1-12 mg/g, 1-10 mg/g, 2-10 mg/g, 2-8 mg/g, 2-6 mg/g, 3-6 mg/g, 0.2-15 mg/g, 0.2-12 mg/g, 0.2-10 mg/g, 0.2-8 mg/g, 0.2-6 mg/g, 0.2-4 mg/g, 0.2-2 mg/g, 0.2-1 mg/g or 0.2-0.8 mg/g in the pharmaceutical composition.

In some embodiments, the pharmaceutically acceptable carrier comprises at least about 50% (w/w), at least about 60% (w/w), at least about 70% (w/w), at least about 80% (w/w), at least about 85% (w/w), at least about 90% (w/w), at least about 95% (w/w), at least about 97% (w/w), at least about 98% (w/w), at least about 99% (w/w) or 100% (w/w) of the lipid.

In some embodiments, the lipid comprises a lipid with a melting point of -20-80°C, -20-10°C or -20-0°C.

In some embodiments, the lipid has a degree of unsaturation of 0-5, 0-4, 0-3, 0-2, 0-1 or 0.

In some embodiments, the lipid comprises a lipid which has a fatty acid carbon chain at a length in a range of 4-24, 4-22, 4-20, 6-20, 6-16, 6-14, 6-13, 6-12, 8-13, 8-12 or 8-10 carbon atoms.

In some embodiments, the lipid comprises a lipid which has a fatty acid chain at a length of 8 and 10, and optionally further comprises a lipid which has the fatty acid carbon chain at a length of 12-22.

In some embodiments, the fatty acid chain in the lipid is a long-chain fatty acid, a medium-chain fatty acid or a short-chain fatty acid.

In some embodiments, the lipid is vegetable oil. In some embodiments, the vegetable oil is olive oil, tea oil, rapeseed oil, peanut oil, soybean oil, corn oil, safflower oil, groundnut oil, sunflower seed oil, canola oil, walnut oil, almond oil, avocado oil, castor oil, coconut oil, cottonseed oil, rice bran oil, sesame oil, refined palm oil or a mixture thereof.

In some embodiments, the lipid is a fatty acid, a fatty acid ester, a fatty alcohol, a lipoid, a paraffin or a mixture thereof.

In some embodiments, the lipoid is a phospholipid, a sucrose ester, a steroid, a fat-soluble vitamin or a mixture thereof.

In some embodiments, the fatty acid ester is a glyceride, an ethylene glycol ester, a propylene glycol ester or a mixture thereof. In some embodiments, the fatty acid ester is a monoester, a diester, a triester or a mixture thereof. In some embodiments, the fatty acid ester comprises glycerides of octanoic acid and/or decanoic acid. In some embodiments, the fatty acid ester is substantially consisting of glycerides of octanoic acid and/or decanoic acid. In some embodiments, the fatty acid ester comprises a medium-chain triglyceride. In some embodiments, the fatty acid ester is a medium-chain triglyceride.

In some embodiments, the pharmaceutically acceptable carrier does not comprise an unsaturated lipid.

In some embodiments, the pharmaceutically acceptable carrier further comprises an antioxidant. In some embodiments, the antioxidant is at an amount of 0.001%-5% (wt), 0.005%-5% (wt), 0.01%-5% (wt), 0.05%-5% (wt), 0.1%-5% (wt), 0.1%-3% (wt), 0.1%-2% (wt), 0.1%-1% (wt), 0.1%-0.8% (wt), 0.1%-0.5% (wt), 0.1%-0.3% (wt), 0.3%-2% (wt), 0.5%-2% (wt), 0.8%-2% (wt) or 1%-2% (wt) based on the weight of the pharmaceutical composition. In some embodiments, the antioxidant is sulfite, bisulfite, pyrosulfite, dithiocarbamate, ascorbic acid, ascorbyl palmitate, hydrocoumarin, vitamin E, ethanolamine, propyl gallate, butylated hydroxyanisole (BHA), butylated hydroxytoluene (BHT), nordihydroguaiaretic acid or glutathione.

In some embodiments, the pharmaceutically acceptable carrier does not comprise an antioxidant.

In some embodiments, the pharmaceutically acceptable carrier further comprises a viscosity modifier, a pH regulator or a flavoring agent.

In some embodiments, the pharmaceutically acceptable carrier further comprises ethanol. In some embodiments, the ethanol is at an amount of 10%-0.1% (v/v). In some embodiments, the ethanol is at an amount of 8%-0.1 % (v/v), 7%-0.1 % (v/v), 6%-0.1 % (v/v), 5%-0.1% (v/v), 4%-0.1 % (v/v), 3%-0.1% (v/v), 2%-0.1% (v/v), 1.5%-0.1% (v/v), 1.2%-0.1% (v/v), 8%-0.3% (v/v), 8%-0.5% (v/v), 8%-0.7% (v/v), 8%-0.9% (v/v), 8%-1% (v/v), 6%-0.3% (v/v), 5%-0.5% (v/v), 4%-0.8% (v/v), 3%-0.9% (v/v) or 2%-1% (v/v).

In some embodiments, the pharmaceutical composition is used for oral, subcutaneous, intramuscular or intravenous administration.

In some embodiments, the pharmaceutical composition is tablets, capsules, suspensions, solutions, semisolid preparations, patches or microneedles.

In some embodiments, the micromolecule PI4KIIIα inhibitor is phenylarsine oxide, the phenylarsine oxide is at an amount of 0.1-20 mg/g in the pharmaceutical composition, and the pharmaceutically acceptable carrier is consisting of a medium-chain triglyceride, consisting of a medium-chain triglyceride and a long-chain triglyceride, or consisting of a medium-chain triglyceride and ethanol.

In some embodiments, phenylarsonic acid is at an amount of less than 5%, 4%, 3%, 2%, 1%, 0.7%, 0.5%, 0.3% or 0.2% in the pharmaceutical composition. In some embodiments, the phenylarsonic acid is at an amount of less than 5%, 4%, 3%, 2%, 1%, 0.7%, 0.5%, 0.3% or 0.2% after the pharmaceutical composition is stored under conditions of 25°C/60%RH for 1 month, 2 months, 3 months, 4 months, 5 months, 6 months, 7 months, 8 months, 9 months, 10 months, 11 months, 1 year, 1.5 years, 2 years or 3 years. In some embodiments, the phenylarsonic acid is at an amount of less than 5%, 4%, 3%, 2%, 1%, 0.7%, 0.5%, 0.3% or 0.2% after the pharmaceutical composition is stored under a condition of 2-8°C for 1 month, 2 months, 3 months, 4 months, 5 months, 6 months, 7 months, 8 months, 9 months, 10 months, 11 months, 1 year, 1.5 years, 2 years or 3 years.

In another aspect, the present disclosure provides a method for preparing the pharmaceutical composition provided herein. The method comprises: mixing the micromolecule PI4KIIIα inhibitor and the pharmaceutically acceptable carrier to obtain a mixture.

In some embodiments, the method comprises: mixing the micromolecule PI4KIIIα inhibitor and the pharmaceutically acceptable carrier through a mechanical force. In some embodiments, the mechanical force is stirring, dispersing, shaking or ultrasonic treatment.

In some embodiments, the method comprises: mixing the micromolecule PI4KIIIα inhibitor and the pharmaceutically acceptable carrier after melting the pharmaceutically acceptable carrier by heating.

In some embodiments, the method further comprises: filtering the mixture.

In another aspect, the present disclosure provides a method for treating a PI4KIIIα-related disease in a subject. The method comprises administrating the pharmaceutical composition provided herein to a subject in need thereof.

In some embodiments, the PI4KIIIα-related disease is Alzheimer's disease.

In some embodiments, the subject is an animal such as a pig, a dog, a monkey, a cat, a mouse, or a rat, or a human.

In another aspect, the present disclosure provides use of the pharmaceutical composition provided herein in the manufacture of a medicament for treating a PI4KIIIα-related disease in a subject.

In still another aspect, the present disclosure provides the pharmaceutical composition provided herein for use in treating a PI4KIIIα-related disease in a subject.

### BRIEF DESCRIPTION OF THE DRAWINGS

FIG. 1 shows the dissolution profiles of the PAO. In the figure, the cumulative dissolution % of a sample at 60 min is shown as zero because of data missing, not indicating that the cumulative dissolution % is zero indeed.
FIG. 2 shows the in vitro release profiles of MCT solution samples.
FIG. 3 shows the in vitro release profiles of glyceryl behenate solid dispersion samples.
FIG. 4 shows the in vitro release profiles of MC suspensions.
FIG. 5 shows the in vitro release profiles of MC+0.1% Tween 80 suspensions.
FIG. 6A shows the blood concentrations of the PAO after intravenous administration of the PAO at 0.1 mg/kg; FIG. 6B shows the blood concentrations of the PAO after oral administration of the PAO at 0.2 mg/kg; and FIG. 6C shows the average blood concentrations of the PAO after intravenous or oral administration.
FIG. 7A shows the blood concentrations of the PAO after intravenous administration of the PAO at 0.1 mg/kg; FIG. 7B shows the blood concentrations of the PAO after oral administration of the PAO at 0.2 mg/kg; and FIG. 7C shows the average blood concentrations of the PAO after intravenous or oral administration.
FIG. 8A shows the blood concentrations of the PAO after oral administration of the PAO in a DMSO solution at 0.1 mg/kg; and FIG. 8B shows the blood concentrations of the PAO after oral administration of the PAO in an MCT solution at 0.1 mg/kg.
FIG. 9 shows the weight changes of female mice 1.5 months after intragastric administration of the PAO in a 0.1% DMSO solution or the PAO in an MCT solution at 1.5 mg/kg/day, where "^{∗}" and "^{∗∗}" represent p value of less than 0.05 and 0.01, respectively.

### DETAILED DESCRIPTION

The following description of the disclosure is merely intended to illustrate various embodiments of the disclosure. The specific embodiments discussed are not to be construed as limitations on the scope of the disclosure. It will be apparent to one skilled in the art that various equivalents, changes, and modifications may be made without departing from the spirit and essence of the disclosure, and it is understood that such equivalent embodiments are to be included herein. All references cited herein, including publications, patents and patent applications are incorporated herein by reference in their entirety.

In an aspect of the present disclosure, provided is a pharmaceutical composition comprising a micromolecule PI4KIIIα inhibitor and a pharmaceutically acceptable carrier. The pharmaceutically acceptable carrier comprises a lipid.

### Micromolecule PI4KIIIα inhibitor

As used herein, the term "micromolecule PI4KIIIα inhibitor" refers to various micromolecule compounds that can reduce, decrease, or eliminate the transcription or translation of a PI4KIIIα gene, and/or the concentration or activity of a PI4KIIIα protein. In some embodiments, the micromolecule PI4KIIIα inhibitor is capable of reducing the activity of the PI4KIIIα by at least 5%, 10%, 20%, 40%, 50%, 80%, 90%, 95% or more.

In some embodiments, the micromolecule PI4KIIIα inhibitor is a micromolecule organic or inorganic compound (e.g., a molecule obtained from an artificially synthesized chemical library and a natural product library). In some embodiments, the micromolecule PI4KIIIα inhibitor has a molecular weight of less than 3,000, 2,500, 2,000, 1,500, 1,000, 900, 800, 700, 600, 500, 400, 300, 250, or 200 Daltons.

In some embodiments, the micromolecule PI4KIIIα inhibitor directly binds to the PI4KIIIα protein. In some embodiments, the micromolecule PI4KIIIα inhibitor specifically binds to the PI4KIIIα protein.

As used herein, the term "specific binding", when used to describe the PI4KIIIα inhibitor, means that the PI4KIIIα inhibitor preferably recognizes the PI4KIIIα protein in a complex mixture, and the binding constant of the inhibitor to the PI4KIIIα protein is at least 2 times as high as that of the inhibitor to other non-specific binding proteins. In certain embodiments, the equilibrium dissociation constant of the PI4KIIIα inhibitor from the PI4KIIIα protein is less than or equal to 10⁻⁵ or 10⁻⁶ M. In certain embodiments, the equilibrium dissociation constant of the PI4KIIIα inhibitor from the PI4KIIIα protein is less than or equal to 10⁻⁶ or 10⁻⁷ M. In certain embodiments, the equilibrium dissociation constant of the PI4KIIIα inhibitor from the PI4KIIIα protein is less than or equal to 10⁻⁷ or 10⁻⁸ M.

In some embodiments, the micromolecule PI4KIIIα inhibitor provided herein is PAO and a derivative of PAO.

As used herein, the term "PAO" refers to a micromolecule compound with an arsenic oxide group and a benzene ring as basic structures. Its specific chemical structure is as follows:

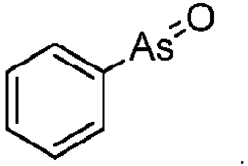

In the present disclosure, the PAO and PI01 are used interchangeably.

As used herein, the term "a derivative of PAO" refers to a class of micromolecule compounds derived from the PAO. These micromolecule compounds have the same basic structures as the PAO (i.e., having an arsenic oxide group and a benzene ring), and can all inhibit PI4KIIIα. In some embodiments, the inhibitory activity of the derivative of PAO on PI4KIIIα is at least 50%, 80%, 90%, 95%, 100%, 120%, 150%, 1 time, 2 times, 3 times, 4 times or more times as high as the inhibitory activity of the PAO. In some embodiments, the solubility of the derivative of PAO in water is 50%-200%, 80%-180%, 90%-150%, 95%-150%, 100-150%, 120%-150%, 80%-150%, 80%-130%, 80%-120% or 90%-110% of the solubility of the PAO in water. In some embodiments, the solubility of the derivative of PAO in the pharmaceutically acceptable carrier provided herein is 50%-200%, 80%-180%, 90%-150%, 95%-150%, 100-150%, 120%-150%, 80%-150%, 80%-130%, 80%-120% or 90%-110% of the solubility of the PAO in the pharmaceutically acceptable carrier provided herein.

In some embodiments, the micromolecule PI4KIIIα inhibitor provided herein has a structure of formula (I) or a pharmaceutically acceptable salt thereof, 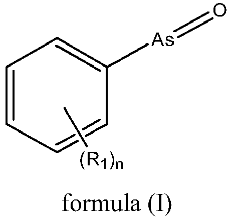 wherein R₁ is each independently selected from H, halogen, nitro, cyano, hydroxyl, amino, carbamoyl, C₁₋₆ alkyl, C₁₋₆ alkoxy, C₁₋₆ haloalkyl, -As(O), -NH-(C₁₋₆ alkyl), N,N-(C₁₋₆ alkyl)₂, -NH-C(O)-R₂, -NH-S(O)₂-R₃, -C(O)OR₄ or heterocyclyl, wherein n is an integer of 0-5, R₂ and R₃ are each independently selected from H, amino, C₁₋₆ alkyl, C₁₋₆ alkoxy, C₁₋₆ haloalkyl, -NH-(C₁₋₆ alkyl), N,N-(C₁₋₆ alkyl)₂, -C(O)OR₄, C₃₋₆ cycloalkyl, 6-12 membered aryl or 3-6 membered heterocyclyl, which are optionally substituted by halogen, nitro, cyano, hydroxyl, amino, carbamoyl, aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, and R₄ is C₁₋₆ alkyl.

In some embodiments, n is 0, 1, 2 or 3. In some embodiments, n is 0, 1 or 2. In some embodiments, n is 0 or 1.

In some embodiments, R₁ is each independently selected from H, halogen, nitro, cyano, hydroxyl, amino, carbamoyl, C₁₋₆ alkyl, C₁₋₆ alkoxy, C₁₋₆ haloalkyl, -As(O), - NH-(C₁₋₆ alkyl), N,N-(C₁₋₆ alkyl)₂ or -C(O)OR₄, where n is an integer of 0-2, and R₄ is C₁₋₆ alkyl.

In some embodiments, R₁ is each independently selected from H, halogen, nitro, cyano, hydroxyl, amino, C₁₋₆ alkyl, C₁₋₆ alkoxy, C₁₋₆ haloalkyl or -As(O), where n is an integer of 0-2.

In some embodiments, R₁ is each independently selected from H, halogen, amino or C₁₋₆ alkoxy, where n is 1.

In some embodiments, R₁ is located at an ortho position or a para position of the -As(O) group. In some embodiments, R₁ is H.

As used herein, the term "substituted", when referring to a chemical group, means that one or more hydrogen atoms of the chemical group are removed and substituted by a substituent.

As used herein, the term "substituent" has the common meaning well known in the art and refers to a chemical moiety that is covalently attached to or fused to a parent group where appropriate.

As used herein, the term "Cₙ-Cₘ" represents a range of the number of carbon atoms, where n and m are integers, and the range of the number of carbon atoms includes endpoints (i.e., n and m) and every integer point therebetween. For example, C₁-C₆ represents a range of 1 to 6 carbon atoms, including 1 carbon atom, 2 carbon atoms, 3 carbon atoms, 4 carbon atoms, 5 carbon atoms and 6 carbon atoms.

As used herein, the term "alkyl", whether used as part of other terms or used alone, refers to a saturated hydrocarbyl group, which may be linear or branched. The term "Cₙ-Cₘ alkyl" refers to an alkyl having n to m carbon atoms. In certain embodiments, the alkyl group includes 1 to 12, 1 to 8, 1 to 6, 1 to 4, 1 to 3, or 1 to 2 carbon atoms. An example of the alkyl group includes, but is not limited to, a chemical group such as methyl, ethyl, *n-*propyl, isopropyl, *n*-butyl, tert-butyl, isobutyl, sec-butyl, 2-methyl-1-butyl, *n*-pentyl, 3-pentyl, *n*-hexyl, 1,2,2-trimethylpropyl, etc.

As used herein, the term "alkenyl", whether used as part of other terms or used alone, refers to an unsaturated hydrocarbyl group, which may be linear or branched and has at least one carbon-carbon double bond. In certain embodiments, the alkenyl group includes 2 to 12, 2 to 10, 2 to 8, 2 to 6, 2 to 5, 2 to 4, or 2 to 3 carbon atoms. In certain embodiments, the alkenyl group can also have 1 to 6, 1 to 5, 1 to 4, 1 to 3, 1 to 2, or 1 carbon-carbon double bond. An example of the alkenyl group includes, but is not limited to, a chemical group such as vinyl, *n*-propenyl, isopropenyl, *n*-butenyl, sec-butenyl, etc.

As used herein, the term "alkynyl", whether used as part of other terms or used alone, refers to an unsaturated alkynyl group, which may be linear or branched and has at least one carbon-carbon triple bond. In certain embodiments, the alkynyl group includes 2 to 12, 2 to 10, 2 to 8, 2 to 6, 2 to 5, 2 to 4, or 2 to 3 carbon atoms. In certain embodiments, the alkynyl group can also have 1 to 6, 1 to 5, 1 to 4, 1 to 3, 1 to 2, or 1 carbon-carbon triple bond. An example of the alkynyl group includes, but is not limited to, a chemical group such as ethynyl, propynyl, butynyl, etc.

As used herein, the term "cycloalkyl" refers to a cyclic alkyl consisting of at least 3 atoms. The term "n-m membered cycloalkyl" refers to a cycloalkyl having n to m ring-forming members. In addition, the ring may also have one or more double bonds, but not a fully conjugated system. In certain embodiments, the cycloalkyl has 3 to 8, 3 to 6, or 4 to 6 ring-forming carbon atoms. An example of the cycloalkyl includes, but is not limited to, cyclopropyl, cyclobutyl, cyclopentyl, etc.

As used herein, the term "heterocyclyl" refers to a cyclyl of which at least one atom in the ring system is a heteroatom and the remaining ring atoms are carbon atoms. The term "n-m membered heterocyclyl" refers to a heterocyclyl having n to m ring-forming members. As used herein, the term "heterocyclyl" includes heteroaryl and heterocycloalkyl. In addition, the ring may also have one or more double bonds. In certain embodiments, the heterocyclyl is a saturated heterocycloalkyl. An example of the heteroatom includes, but is not limited to, oxygen, sulfur, nitrogen, phosphorus, etc.

As used herein, the term "heterocycloalkyl" refers to a cycloalkyl of which at least one atom in the ring system is a heteroatom and the remaining ring atoms are carbon atoms. The term "n-m membered heterocycloalkyl" refers to a heterocycloalkyl having n to m ring-forming members. In addition, the ring may also have one or more double bonds, but not a fully conjugated system. In certain embodiments, the heterocycloalkyl is a saturated heterocycloalkyl. An example of the heteroatom includes, but is not limited to, oxygen, sulfur, nitrogen, phosphorus, etc. In certain embodiments, the heterocycloalkyl has 3 to 8, 3 to 6, or 4 to 6 ring-forming carbon atoms. An example of the heterocycloalkyl includes, but is not limited to, azetidine, aziridine, pyrrolidinyl, piperidinyl, piperazinyl, morpholinyl, thiomorpholine, homopiperazine, etc.

As used herein, the term "aryl" or "aromatic group", whether used as part of other terms or used alone, refers to a single-carbocycle or multi-carbocycle cyclic group having alternate double bonds and single bonds between ring-forming carbon atoms. The term "Cₙ-Cₘ aryl" refers to an aryl having n to m ring-forming carbon atoms. In certain embodiments, an aryl ring system has 6 to 12, 6 to 10, or 6 to 8 carbon atoms in one or more rings. In certain embodiments, the aryl ring system has 2 or more rings fused together. An example of the aryl group includes, but is not limited to, a chemical group such as phenyl, naphthyl, tetrahydronaphthyl, indanyl, indenyl, etc.

As used herein, the term "heteroaryl" refers to an aryl group of which at least one ring atom in the aromatic ring is a heteroatom and the remaining ring atoms are carbon atoms. The term "n-m membered heteroaryl" refers to a heteroaryl having n to m ring-forming members. An example of the heteroatom includes, but is not limited to, oxygen, sulfur, nitrogen, phosphorus, etc. In certain embodiments, the heteroaryl may have 5 to 10, 5 to 8, or 5 to 6 ring-forming members. In certain embodiments, the heteroaryl is a 5 or 6 membered heteroaryl. An example of the heteroaryl includes, but is not limited to, furyl, thienyl, pyridyl, quinolinyl, pyrrolyl, N-lower alkylpyrrolyl, pyridyl-N-oxide, pyrimidinyl, pyrazinyl, imidazolyl, indolyl, etc.

As used herein, the term "alkoxy", whether used as part of other terms or used alone, refers to a group of formula "-O-alkyl". The term "Cₙ-Cₘ alkoxy" means that an alkyl moiety of the alkoxy has n to m carbon atoms. In certain embodiments, the alkyl moiety has 1 to 6, 1 to 4, or 1 to 3 carbon atoms. An example of the alkoxy group includes, but is not limited to, a chemical group such as methoxy, ethoxy, propoxy (e.g., *n*-propoxy and isopropoxy), *t*-butoxy, etc.

As used herein, the term "haloalkyl", whether used as part of other terms or used alone, refers to a group of formula "-alkyl-X", where X is halogen, an atom selected from fluorine, chlorine, bromine and iodine. The term "Cₙ-Cₘ haloalkyl" means that an alkyl moiety of the haloalkyl has n to m carbon atoms. In certain embodiments, the alkyl moiety has 1 to 6, 1 to 4, or 1 to 3 carbon atoms. An example of the haloalkyl group includes, but is not limited to, a chemical group such as halomethyl, haloethyl, halopropyl (e.g., *n*-halopropyl and isohalopropyl), *t*-halobutyl, etc.

As used herein, the term "n membered" is usually used with a ring system to describe the number of ring-forming atoms in the ring system, where n is an integer. For example, piperidinyl is an example of a 6 membered heterocycloalkyl ring, pyrazolyl is an example of a 5 membered heteroaryl ring, pyridyl is an example of a 6 membered heteroaryl ring, and 1,2,3,4-tetrahydro-naphthalene is an example of a 10 membered aryl.

As used herein, the term "halogen" refers to an atom selected from fluorine, chlorine, bromine and iodine.

As used herein, the term "cyano" refers to a group of formula "-CN".

As used herein, the term "hydroxyl" refers to a group of formula "-OH".

As used herein, the term "nitro" refers to a group of formula "-NO₂".

As used herein, the term "amino" refers to a group of formula "-NH₂".

As used herein, the term "carbamoyl" refers to a group of formula "-HNCONH₂".

As used herein, the term "compound" is intended to include all stereoisomers (e.g., enantiomers and diastereomers), geometric isomers, tautomers and isotopes of the shown structure.

The compound provided herein may be asymmetric (e.g., having one or more stereocenters). Unless otherwise indicated, all the stereoisomers, such as enantiomers and diastereomers, are intended to be included. The compound provided herein including asymmetrically substituted carbon atoms may be separated in an optically activated or racemic form. Methods to prepare the optically active form from starting materials that are not optically active are well known in the art, such as by resolution of racemic mixtures or by stereoselective synthesis. Various geometric isomers, such as olefins, carbon-carbon double bonds and the like, may also exist in the compound provided herein, and all of these stable isomers have been considered in the present disclosure. The present disclosure describes cis and trans geometric isomers of the compound, which may be separated as a mixture of isomers or as individual isomers.

In certain embodiments, the compound provided herein has a (R)-configuration. In certain embodiments, the compound provided herein has a (*S*)-configuration.

The racemic mixture of the compound may be resolved by any one of multiple methods well known in the art. An exemplary method includes fractional crystallization using a chiral resolving acid which is an optically active salt-forming organic acid. Suitable resolving reagents for the fractional recrystallization method are, for example, optically active acids (e.g., D and L forms of tartaric acid, diacetyltartaric acid, dibenzoyltartaric acid, mandelic acid, malic acid, lactic acid) or various optically active camphorsulfonic acids. Other resolving reagents suitable for the fractional crystallization method include stereoisomerically pure forms of N-methylbenzylamine, 2-phenylglycinol, norephedrine, ephedrine, *N*-methylephedrine, cyclohexylethylamine, 1,2-diaminocyclohexane, etc.

The racemic mixture may also be resolved by elution on a column provided with an optically active resolving reagent (e.g., dinitrobenzoylphenylglycine). A suitable elution solvent composition may be determined by a person skilled in the art.

The compound provided herein also includes tautomeric forms. The tautomeric forms are caused by the interconversion between a single bond and an adjacent double bond both accompanied by the migration of protons. The tautomeric forms include tautomers of protons in an isomeric protonated state with the same chemical formula and total charge. Examples of the proton tautomers include a keto-enol pair, an amide-imidic acid pair, a lactam-lactim pair, an enamine-imine pair, and an annular form in which protons can occupy two or more positions of a heterocyclic system, such as 1H- and 3H-imidazole, 1H-, 2H- and 4H-1,2,4-triazole, 1H- and 2H-isoindole, and 1H- and 2H-pyrazole. The tautomeric forms can be balanced or sterically locked into one form through appropriate substitution.

The compound provided herein can also include all isotopes of atoms existing in intermediate or final compounds. The isotopes include those atoms with the same atomic number but different mass numbers. For example, isotopes of hydrogen include protium, deuterium and tritium.

In certain embodiments, the micromolecule compound provided herein may be obtained by organic synthesis. The compound provided herein, including salts, esters, hydrates or solvates thereof, may be prepared by any well-known organic synthesis technology and may be synthesized according to many possible synthesis routes.

The reaction for preparing the compound provided herein may be carried out in a suitable solvent, and a person skilled in the field of organic synthesis can easily select the solvent. The suitable solvent cannot substantially react with starting materials (reactants), intermediates or products at the reaction temperature (for example, the temperature may range from a freezing temperature of the solvent to a boiling temperature of the solvent). A given reaction may be carried out in one solvent or a mixture of more than one solvent. According to specific reaction steps, a person skilled in the art can select suitable solvents for the specific reaction steps.

The preparation of the compound provided herein may involve the protection and deprotection of various chemical groups. A person skilled in the art can easily determine whether protection and deprotection are needed and select suitable protective groups. Chemistry of the protective groups can be found in, for example, T. W. Greene and P. G. M. Wuts, Protective Groups in Organic Synthesis, 3rd Ed., Wiley & Sons, Inc., New York (1999), the entire contents of which are incorporated into the present disclosure by reference.

The reaction may be monitored according to any suitable method well known in the art. For example, the formation of products may be monitored by using spectroscopy, such as nuclear magnetic resonance spectroscopy (e.g., ¹H or ¹³C), infrared spectroscopy, spectrophotometry (e.g., UV-visible), mass spectrometry; or by using chromatography, such as high performance liquid chromatography (HPLC), liquid chromatography-mass spectrometry (LCMS) or thin-layer chromatography (TLC). A person skilled in the art may purify the compound by many methods, including high performance liquid chromatography (HPLC) (see, for example, "Preparative LC-MS Purification: Improved Compound Specific Method Optimization" Karl F. Blom, Brian Glass, Richard Sparks, Andrew P. Combs J. Combi. Chem. 2004, 6(6), 874-883, the entire contents of which are incorporated into the present disclosure by reference) and normal-phase silica gel column chromatography.

In certain embodiments, the micromolecule compound provided herein may be commercially available.

In some embodiments, the micromolecule PI4KIIIα inhibitor provided herein is at an amount of 0.01-20 mg/g, 0.05-20 mg/g, 0.1-20 mg/g, 0.2-20 mg/g, 0.5-20 mg/g, 0.8-20 mg/g, 1-20 mg/g, 1-18 mg/g, 1-16 mg/g, 1-14 mg/g, 1-12 mg/g, 1-10 mg/g, 2-10 mg/g, 2-8 mg/g, 2-6 mg/g, 2-5 mg/g, 2-4 mg/g, 2-3 mg/g, 3-6 mg/g, 0.2-15 mg/g, 0.2-12 mg/g, 0.2-10 mg/g, 0.2-8 mg/g, 0.2-6 mg/g, 0.2-4 mg/g, 0.2-2 mg/g, 0.2-1 mg/g or 0.2-0.8 mg/g in the pharmaceutical composition.

### Pharmaceutically acceptable carrier

As used herein, the term "pharmaceutically acceptable" refers to those compounds, materials, compositions and/or dosage forms that are suitable for use in contact with human and animal tissues within the scope of reasonable medical judgment without excessive toxicity, irritation, allergic reaction or other problems or complications, and have a reasonable benefit/risk ratio. In certain embodiments, the pharmaceutically acceptable compounds, materials, compositions and/or dosage forms refer to those used for animals (more particularly for humans) approved by regulatory authorities (e.g., U.S. Food and Drug Administration, State Food and Drug Administration or European Medicines Agency) or listed in widely accepted pharmacopoeia (e.g., U.S. Pharmacopoeia, Pharmacopoeia of the People's Republic of China or European Pharmacopoeia).

Pharmaceutically acceptable carriers that may be used in the pharmaceutical composition provided herein include, but are not limited to, for example pharmaceutically acceptable liquid, gel or solid vehicles, aqueous media (e.g., sodium chloride injection, Ringer's solution injection, isotonic glucose injection, sterile water injection, or glucose and lactated Ringer's injection), non-aqueous media (e.g., plant-derived nonvolatile oil, cottonseed oil, corn oil, sesame oil, peanut oil or medium/medium-to-long-chain glyceride, such as medium-chain triglyceride), antimicrobial substances, isotonic substances (e.g., sodium chloride or glucose), buffers (e.g., phosphate or citrate buffers), antioxidants (e.g., sodium bisulfate), anesthetics (e.g., procaine hydrochloride), suspending agents/dispersing agents (e.g., sodium carboxymethyl cellulose, hydroxypropyl methylcellulose, or polyvinylpyrrolidone), chelating agents (e.g., EDTA (ethylenediaminetetraacetic acid) or EGTA (ethylene glycol bis(2-aminoethyl ether)tetraacetic acid)), emulsifiers (e.g., Polysorbate 80 (Tween-80)), diluents, adjuvants, or nontoxic auxiliary substances, other components well known in the art, or various combinations of the above. Suitable components may include, for example, fillers, binders, disintegrants, buffers, preservatives, lubricants, flavoring agents, thickeners, colorants or emulsifiers.

In some embodiments, the pharmaceutically acceptable carrier provided herein further includes an antioxidant, such as sulfite, bisulfite, pyrosulfite, dithiocarbamate, ascorbic acid, ascorbyl palmitate, hydrocoumarin, vitamin E, ethanolamine, propyl gallate, butylated hydroxyanisole (BHA), butylated hydroxytoluene (BHT), nordihydroguaiaretic acid or glutathione. In some embodiments, the antioxidant provided herein is at an amount of 0.001%-5% (wt), 0.005%-5% (wt), 0.01%-5% (wt), 0.05%-5% (wt), 0.1%-5% (wt), 0.1%-3% (wt), 0.1%-2% (wt), 0.1%-1% (wt), 0.1%-0.8% (wt), 0.1%-0.5% (wt), 0.1%-0.3% (wt), 0.3%-2% (wt), 0.5%-2% (wt), 0.8%-2% (wt) or 1%-2% (wt) based on the weight of the pharmaceutical composition.

In some embodiments, the pharmaceutically acceptable carrier provided herein does not comprise antioxidants.

In some embodiments, the pharmaceutically acceptable carrier provided herein further comprises a viscosity modifier, a pH regulator or a flavoring agent.

The pharmaceutical composition provided herein may be used in administration routes well known in the art, such as injection administration (e.g., subcutaneous injection, intraperitoneal injection, intravenous injection (including intravenous drip or intravenous infusion), intramuscular injection or intradermal injection) or non-injection administration (e.g., oral administration, nasal administration, sublingual administration, rectal administration or external administration). In some embodiments, the pharmaceutical composition provided herein is used for oral, subcutaneous, intramuscular or intravenous administration.

In some embodiments, the pharmaceutical composition provided herein may be prepared into dosage forms for oral administration (including but not limited to capsules, tablets, pills, aqueous suspensions or solutions), dosage forms for injection administration (including but not limited to solutions, emulsions, liposomes, powder injections), suppositories for rectal administration, and dosage forms for topical administration (including but not limited to ointments, pastes, creams, lotions, gels, powder, solutions, sprays, inhalants or patches), etc.

In some embodiments, the pharmaceutical composition provided herein is tablets, capsules, suspensions, solutions, semisolid preparations, patches or microneedles.

In some embodiments, the pharmaceutical composition provided herein is an oral liquid. As used herein, the term "oral liquid" is a liquid dosage form for oral administration, which includes (but is not limited to) pharmaceutically acceptable emulsions, microemulsions, solutions, suspensions, syrups and elixirs. In addition to active compounds, the liquid dosage form may include commonly used inert diluents (e.g., water or other solvents), solubilizers, emulsifiers, wetting agents, emulsifiers and suspending agents, sweetening agents, flavoring agents and fragrances. In some embodiments, the oral liquid is in the form of a solution. In some embodiments, the oral liquid may be diluted with a diluent before being administered to a patient. In some embodiments, the diluent is vegetable oil, or an aqueous solution having a certain flavoring effect, such as soda water, fruit juice, etc.

In some embodiments, the pharmaceutical composition provided herein is an injection.

As used herein, the term "injection" refers to a preparation for injection, in which medicaments are formulated into solutions (aqueous or non-aqueous), suspensions or emulsions and filled into ampoules or multi-dose containers. The injection, such as a sterile injectable aqueous or oily suspension, may be formulated according to known technologies using suitable dispersing agents or wetting agents, suspending agents and emulsifiers. In some embodiments, the pharmaceutical composition provided herein is an oily injection. In some embodiments, the pharmaceutical composition provided herein is an injection including the lipid provided herein. In some embodiments, the pharmaceutical composition provided herein is an injection including mono-/di-glycerides of octanoic/decanoic acid or medium-chain triglycerides. In some embodiments, the pharmaceutical composition provided herein is prepared into a pre-filled dosage form.

In some embodiments, the pharmaceutical composition provided herein is patches.

As used herein, the term "patch" refers to a flaky preparation which is made from active pharmaceutical ingredients and suitable materials and may produce systemic or topical effects when pasted on the skin. The patch is consisting of a backing layer, a medicament-containing matrix, a pressure-sensitive adhesive and an anti-sticking layer to be removed before use. The patch may be used on intact skin surfaces, and may be also used on diseased or incomplete skin surfaces. The patch which is used on the intact skin surfaces and can diffuse medicaments through the skin into the blood circulation system is known as a transdermal patch. The action time of the transdermal patch is determined by its medicament content and release rate. The patch may be classified into an adhesive dispersion type, a reservoir type and a peripheral adhesive type. In some embodiments, the pharmaceutical composition provided herein is a patch including the lipid provided herein. In some embodiments, the pharmaceutical composition provided herein is a patch including mono-/diglycerides of octanoic/decanoic acid or medium-chain triglycerides.

In some embodiments, the pharmaceutical composition provided herein is microneedles.

As used herein, the term "microneedle" refers to a preparation having a microneedle array that can pierce the stratum corneum to facilitate transdermal delivery of therapeutic agents. In some embodiments, the microneedle has a microneedle array with a height of 300 to 1,000 µm. The microneedle used herein may be made of a material including resin or other polymer materials, ceramics or metals. In addition, the material of the microneedle is preferably a material including thermoplastic resin, and more preferably a material including biodegradable thermoplastic resin. In some embodiments, the pharmaceutical composition provided herein is a microneedle including the lipid provided herein. In some embodiments, the pharmaceutical composition provided herein is a microneedle including mono-/di-glycerides of octanoic/decanoic acid or medium-chain triglycerides. In some embodiments, the pharmaceutical composition provided herein and the microneedle are prepared separately, but used in combination. In some embodiments, the pharmaceutical composition provided herein is used before or after the microneedle, for example, the microneedle is firstly applied to the skin of a patient, and then the pharmaceutical composition provided herein is applied to the same site; alternatively the pharmaceutical composition provided herein is firstly applied to the skin of the patient, and then the microneedle is applied to the same site.

### Lipid

In some embodiments, the pharmaceutically acceptable carrier provided herein includes a lipid.

As used herein, the term "lipid" refers to an ester and derivatives thereof formed by the reaction of a fatty acid and an alcohol. It is a type of compounds generally insoluble in water but soluble in fat-soluble solvents. It may be synthetic, semisynthetic or naturally occurring, including a fat, a phospholipid, a glycolipid, a cholesterol, a cholesterol ester, etc.

In some embodiments, the pharmaceutically acceptable carrier provided herein includes at least about 50% (w/w), at least about 60% (w/w), at least about 70% (w/w), at least about 80% (w/w), at least about 85% (w/w), at least about 90% (w/w), at least about 95% (w/w), at least about 97% (w/w), at least about 98% (w/w), at least about 99% (w/w) or 100% (w/w) of the lipid.

In some embodiments, the lipid provided herein includes a lipid with a melting point of -20-80°C, -20-10°C or -20-0°C. In some embodiments, the lipid provided herein includes a lipid which is a liquid at room temperature. In some embodiments, the lipid provided herein is consisting of a lipid with a melting point of -20-0°C.

As used herein, the term "melting point" refers to a temperature at which the solid state and the liquid state of a substance are in equilibrium under a certain pressure, that is, at this pressure and this melting point temperature, the chemical potential of a substance in the solid state is equal to that in the liquid state. When the substance is pure, it generally has a fixed melting point, that is, under a certain pressure, the temperature difference from initial melting to full melting (the range is known as a melting range) does not exceed 0.5-1°C. The melting point may be measured by conventional methods in the art, including but not limited to capillary measurement, microscope hot plate measurement, automatic melting point measurement, etc. In some embodiments, the melting point provided herein is measured under normal pressure.

In some embodiments, the lipid provided herein has a degree of unsaturation of 0-5, 0-4, 0-3, 0-2, 0-1 or 0. In some embodiments, the lipid provided herein has a degree of unsaturation of 0 or 1. In some embodiments, the lipid provided herein has a degree of unsaturation of 0.

As used herein, the term "degree of unsaturation", also known as an index of hydrogen deficiency or a ring-plus-double-bond index, is a quantitative indicator of the degree of unsaturation of an organic molecule, that is, for every 2 hydrogen atoms reduced in the organic molecule as compared with an open-chain alkane with the same number of carbon atoms, the degree of unsaturation of the organic substance is increased by 1. In general, the degree of unsaturation is represented by a Greek letter Ω. The degree of unsaturation may help to determine how many rings (1 degree of unsaturation), double bonds (1 degree of unsaturation) and triple bonds (2 degrees of unsaturation) a compound has. In some embodiments, the degree of unsaturation provided herein excludes the degree of unsaturation resulting from rings.

According to the degree of saturation, the lipid can be divided into two classes, namely a saturated lipid and an unsaturated lipid. According to the degree of unsaturation, the unsaturated lipid is further divided into a monounsaturated lipid and a polyunsaturated lipid. The monounsaturated lipid has only one double bond in the molecular structure; and a polyunsaturated fatty acid has two or more double bonds in the molecular structure.

In some embodiments, the pharmaceutically acceptable carrier provided herein dose not comprise unsaturated lipids.

In some embodiments, the lipid provided herein includes a lipid which has a fatty acid carbon chain at a length in a range of 4-24, 4-22, 4-20, 6-20, 6-16, 6-14, 6-13, 6-12, 8-13, 8-12 or 8-10 carbon atoms. In some embodiments, the lipid provided herein includes a lipid which has a fatty acid chain at a length of 8 and 10, and optionally further includes a lipid which has the fatty acid carbon chain at a length of 12-22.

As used herein, the term "fatty acid carbon chain length" refers to the number of carbon atoms in a carbon chain in a fatty acid of the lipid.

In some embodiments, the fatty acid chain in the lipid is a long-chain fatty acid, a medium-chain fatty acid or a short-chain fatty acid. In some embodiments, the pharmaceutically acceptable carrier provided herein is consisting of a medium-chain triglyceride, or consisting of a mixture of a medium-chain triglyceride and a long-chain triglyceride.

As used herein, the term "long-chain fatty acid", also known as a higher fatty acid, refers to a fatty acid with more than 12 carbon atoms on a carbon chain. The long-chain fatty acid mainly exists in a natural fat and is a main component of the fat. There are many kinds of long-chain fatty acids in the natural fat. Common ones are palmitic acid (hexadecanoic acid), stearic acid (octadecanoic acid) and oleic acid (octadecene-[9]-acid).

As used herein, the term "medium-chain fatty acid" refers to a fatty acid with 6-12 carbon atoms on a carbon chain, and main components are octanoic acid (C8) and decanoic acid (C10).

As used herein, the term "short-chain fatty acid", also known as a volatile fatty acid, refers to an organic fatty acid with 2-6 carbon atoms on a carbon chain, mainly including acetic acid, propionic acid, isobutyric acid, butyric acid, isovaleric acid and valeric acid.

In some embodiments, the lipid provided herein is vegetable oil.

As used herein, the term "vegetable oil" is a compound formed by esterification of an unsaturated fatty acids and a glycerol. The vegetable oil may be oil obtained from fruits, seeds and germ of plants, such as peanut oil, soybean oil, linseed oil, castor oil, rapeseed oil, etc. A main component of the vegetable oil is an ester generated by a linear higher fatty acid and a glycerol. In addition, the vegetable oil may further include vitamins E, K, minerals such as calcium, iron, phosphorus, potassium, fatty acids, etc.

In some embodiments, the vegetable oil provided herein is olive oil, tea oil, rapeseed oil, peanut oil, soybean oil, corn oil, safflower oil, groundnut oil, sunflower seed oil, canola oil, walnut oil, almond oil, avocado oil, castor oil, coconut oil, cottonseed oil, rice bran oil, sesame oil, refined palm oil or a mixture thereof.

In some embodiments, the lipid provided herein is a fatty acid, a fatty acid ester, a fatty alcohol, a lipoid, a paraffin or a mixture thereof.

In some embodiments, the lipid provided herein is a fatty acid ester. In some embodiments, the fatty acid ester provided herein is a glyceride, an ethylene glycol ester, a propylene glycol ester or a mixture thereof. In some embodiments, the fatty acid ester provided herein is a monoester, a diester, a triester or a mixture thereof. In some embodiments, the fatty acid ester provided herein is glycerides of octanoic acid and/or decanoic acid. In some embodiments, the lipid provided herein is mono-/di-glycerides of octanoic/decanoic acid or medium-chain triglycerides.

As used herein, the term "medium-chain triglyceride (MCT)" refers to triglycerides of fatty acids with a length of 6 to 12 carbon atoms (including one or more of hexanoic acid, octanoic acid, decanoic acid and lauric acid). The medium-chain triglyceride has a low freezing point, is a liquid at room temperature and has low viscosity. In some embodiments, the medium-chain triglyceride provided herein is extracted from dry hard parts of endosperms of coconuts (e.g., *Cocos nucifera* L.) or oil palms (e.g., *Elaeis guineenis Jacq).* A typical medium-chain triglyceride refers to a saturated octanoic acid triglyceride or a saturated decanoic acid triglyceride or a saturated octanoic acid-decanoic acid mixed triglyceride. In some embodiments, the medium-chain triglyceride provided herein meets the standards for a medium-chain triglyceride in widely accepted pharmacopoeia (e.g., U.S. Pharmacopoeia, Pharmacopoeia of the People's Republic of China or European Pharmacopoeia). In some embodiments, the medium-chain triglyceride provided herein is MIGLYOL^{®}812N medium-chain triglyceride.

### Preparation method of pharmaceutical composition

The pharmaceutical composition provided herein may be prepared by conventional methods in the art.

In another aspect, the present disclosure provides a method for preparing the pharmaceutical composition provided herein. The method comprises: mixing the micromolecule PI4KIIIα inhibitor and the pharmaceutically acceptable carrier to obtain a mixture.

In some embodiments, the method comprises: mixing the micromolecule PI4KIIIα inhibitor and the pharmaceutically acceptable carrier through a mechanical force. In some embodiments, the mechanical force is stirring, dispersing, shaking or ultrasonic treatment. In some embodiments, the action time of the mechanical force is 5 hours, 4 hours, 3 hours, 2 hours, 1 hour, 50 minutes, 40 minutes, 30 minutes, 20 minutes or 10 minutes, or a range between any two time points mentioned above. In some embodiments, heating is performed simultaneously in the mixing process. In some embodiments, the heating temperature is 30-80°C, 35-80°C, 40-80°C, 40-70°C, 40-60°C, 45-55°C or 55°C.

In other embodiments, the method comprises: mixing the micromolecule PI4KIIIα inhibitor and the pharmaceutically acceptable carrier after melting the pharmaceutically acceptable carrier by heating.

In some embodiments, the method further comprises: filtering the mixture. In some embodiments, the undissolved micromolecule PI4KIIIα inhibitor is removed by the filtering. In some embodiments, a filtering device used in the filtering substantially does not adsorb the micromolecule PI4KIIIα inhibitor, for example, it adsorbs less than about 1%, 2%, 3%, 5%, 8%, 10%, 12%, 15% or 20% of the micromolecule PI4KIIIα inhibitor in the mixture.

### Disease treatment method and medical use

Another aspect of the present disclosure relates to a method for treating a PI4KIIIα-related disease in a subject. The method comprises administrating the pharmaceutical composition provided herein to a subject in need thereof.

In certain embodiments, the pharmaceutical composition provided herein includes a therapeutically effective amount of the micromolecule PI4KIIIα inhibitor.

As used herein, the term "therapeutically effective amount" refers to an amount of medicaments that may alleviate or eliminate a disease or symptom of a subject or may prophylactically inhibit or avoid the occurrence of the disease or symptom. The therapeutically effective amount may be an amount of medicaments that may alleviate one or more diseases or symptoms of a subject to a certain degree; an amount of medicaments that may partially or completely restore one or more physiological or biochemical parameters related to causes of the diseases or symptoms to normal; and/or an amount of medicaments that may reduce the possibility of occurrence of the diseases or symptoms.

A therapeutically effective dose of the micromolecule PI4KIIIα inhibitor provided herein depends on many factors well known in the art, such as weight, age, past medical history, treatment being currently received, health status of the subject, and intensity, allergy, hypersensitivity and side effects of medicament interaction, as well as administration routes and degree of disease development. Those skilled in the art (e.g., doctors or veterinarians) may reduce or increase the dose according to these or other conditions or requirements accordingly.

As used herein, the term "subject" may include a human and a non-human animal. The non-human animal includes all vertebrates such as a mammal and a non-mammal. The "subject" may also be a farm animal (e.g., a cow, a pig, a sheep, a chicken, a rabbit or a horse), or a rodent (e.g., a rat or a mouse), or a primate (e.g., a gorilla or a monkey), or a domestic animal (e.g., a dog or a cat). The "subject" may be male or female, or it may be of different ages. A human "subject" may be a Caucasian, an African, an Asian, a Semite, or other races, or a hybrid of different races. The human "object" may be an elder, an adult, a teenager, a child or an infant.

In some embodiments, the subject provided herein is an animal such as a pig, a dog, a monkey, a cat, a mouse, or a rat, or a human.

As used herein, the term "PI4KIIIα-related disease" refers to diseases associated with abnormal cellular reactions mediated by a PI4KIIIα protein kinase. In some embodiments, the PI4KIIIα-related disease provided herein is Alzheimer's disease.

The present disclosure further relates to use of the pharmaceutical composition provided herein in the manufacture of a medicament for treating a PI4KIIIα-related disease in a subject and the pharmaceutical composition provided herein for use in treating a PI4KIIIα-related disease in a subject.

### Examples

### Example 1: Stability and solubility of PAO in different vegetable oils

### 1.1 Investigation on dissolution rate of PAO in vegetable oil

Supersaturated PAO vegetable oil solutions were respectively formulated, allowed to stand at room temperature, and sampled at different time points to measure the dissolution rates.

**Table 1: Formulation composition of PAO in vegetable oil in experiment of dissolution rate**

| Formulation | | |
|---|---|---|
| Names of raw and auxiliary materials | PAO | Vegetable oil |
| Ratio | 1 | 100 |
| Theoretical weight | 50 mg | 5 g |

Methods: 50 mg of PAO was respectively weighed and placed into 40 ml vials, and 5 g of corresponding vegetable oil (soybean oil, sesame oil and tea oil) was respectively added. The mixture was stirred on a magnetic stirrer, and sampled at 0.5 h, 1 h, 2.5 h, 4 h and 24 h, respectively. After centrifugal filtration (12,000 rpm), the content was measured by HPLC.

The conditions of the HPLC are shown in Table 2 below:

**Table 2: HPLC conditions**

| **Chromatographic column** | Waters XBridge C18, 5µm 4.6^{∗}250 mm | | |
|---|---|---|---|
| **Wavelength:** | 214 nm | **Column oven** | 25°C |
| **Flow rate** | 1.0 mL/min | | |
| **Mobile phase** | A: 0.5% TFA aqueous solution | | |
| | B: MEOH:ACN=1:1 | | |
| **Gradient elution** | Time (min) | B% | |
| | 0 | 10 | |
| | 7 | 10 | |
| | 30 | 80 | |
| | 35 | 80 | |
| | 40 | 100 | |
| | 50 | 100 | |
| | 50.1 | 10 | |
| | 55 | 10 | |

Unless otherwise specified, the HPLC conditions in all examples of the present disclosure are the same as those mentioned above.

Results:

**Table 3: Results of 24 h dissolution rates for three vegetable oils**

| Matrix | Time | Concentration (mg/ml) |
|---|---|---|
| Tea oil | 0.5 h | 2.51 |
| | 1 h | 3.97 |
| | 2.5 h | 3.81 |
| | 4 h | 3.87 |
| | 24 h | 3.76 |
| Sesame oil | 0.5 h | 3.48 |
| | 1 h | 4.33 |
| | 2.5 h | 4.11 |
| | 4 h | 4.58 |
| | 24 h | 4.31 |
| Soybean oil | 0.5 h | 3.97 |
| | 1 h | 4.81 |
| | 2.5 h | 5.01 |
| | 4 h | 4.97 |
| | 24 h | 4.47 |

Analysis: From the above experimental results, it can be known that in the three vegetable oils, the PAO substantially reached the state of dissolution equilibrium at 1 h. However, the concentration of the PAO in all the three vegetable oils decreased to a certain extent at 24 h.

### 1.2 Investigation on stability of PAO in vegetable oil

Vegetable oil solutions of the PAO were respectively formulated, and allowed to stand. Samples were taken at 0 h, 2 h, 4 h, 20 h, and 48 h respectively to investigate the content and related substances.

**Table 4: Formulation composition of PAO in vegetable oil in experiment of stability**

| Formulation | | |
|---|---|---|
| Names of raw and auxiliary materials | PAO | Vegetable oil |
| Ratio | 1 | 500 |
| Theoretical weight | 20 mg | 10 g |

Methods: 20 mg of PAO was respectively weighed and placed into 20 ml vials, and 10 g of corresponding vegetable oil (tea oil, sesame oil and soybean oil) was added. The mixture was stirred on a magnetic stirrer at room temperature for 30 minutes, and filtered through a 0.22 µm filter. The filtrate was collected, allowed to stand at room temperature, and diluted with isopropanol at 0 h, 2 h, 4 h, 20 h or 48 h respectively. The stability was investigated through HPLC detection.

Results:

**Table 5: Experimental results of stability of PAO in vegetable oil**

| Matrix | Time | RT | % Impurities (area normalization method) | % Total impurities (area normalization method) | Appearance | Dilution fold | Concentration (mg/ml) | % API residue ratio |
|---|---|---|---|---|---|---|---|---|
| Tea oil | 0 h | 6.622 | 0.326 | 0.33 | Clear oily liquid | 7.55 | 0.8361 | - |
| | | 15.545 | 99.674 | | | | | |
| | 2 h | 6.558 | 0.705 | 0.70 | | 7.46 | 0.8505 | 101.72 |
| | | 15.574 | 99.295 | | | | | |
| | 4 h | 6.531 | 1.012 | 1.01 | | 8.07 | 0.8711 | 104.19 |
| | | 15.539 | 98.988 | | | | | |
| | 20 h | 6.422 | 1.798 | 1.80 | | 7.87 | 0.7965 | 95.26 |
| | | 15.407 | 98.203 | | | | | |
| | 48 h | 6.137 | 4.469 | 4.47 | | 7.85 | 0.5856 | 70.04 |
| | | 14.941 | 95.531 | | | | | |
| | 7 d | 6.137 | 2.878 | 2.88 | | 7.84 | 0.7143 | 85.43 |
| | | 15.732 | 97.122 | | | | | |
| Sesame oil | 0 h | 6.586 | 1.482 | 1.58 | Clear oily liquid | 7.75 | 0.8868 | - |
| | | 15.549 | 98.422 | | | | | |
| | | 29.684 | 0.096 | | | | | |
| | 2 h | 6.527 | 4.616 | 5.27 | | 7.82 | 0.7045 | 79.44 |
| | | 15.571 | 94.726 | | | | | |
| | | 27.957 | 0.162 | | | | | |
| | | 29.681 | 0.496 | | | | | |
| | 4 h | 6.491 | 8.065 | 9.55 | Turbid, with production of particles | 7.78 | 0.4931 | 55.60 |
| | | 15.525 | 90.451 | | | | | |
| | | 27.918 | 0.663 | | | | | |
| | | 29.638 | 0.822 | | | | | |
| | 20 h | 6.405 | 22.225 | 31.15 | | 7.91 | 0.1952 | 22.01 |
| | | 15.410 | 68.849 | | | | | |
| | | 20.860 | 1.025 | | | | | |
| | | 27.805 | 6.829 | | | | | |
| | | 29.528 | 1.072 | | | | | |
| Soybean oil | 0 h | 6.622 | 0.316 | 0.32 | Clear oily liquid | 8.27 | 1.3081 | - |
| | | 15.960 | 99.684 | | | | | |
| | 2 h | 6.576 | 0.318 | 0.32 | | 8.20 | 1.3315 | 101.79 |
| | | 15.573 | 99.682 | | | | | |
| | 4 h | 6.587 | 0.322 | 0.32 | | 8.14 | 1.2483 | 95.43 |
| | | 15.558 | 99.678 | | | | | |
| | 20 h | 6.431 | 1.366 | 1.37 | | 8.41 | 1.1416 | 87.27 |
| | | 15.406 | 98.634 | | | | | |
| | 48 h | 6.622 | 8.492 | 8.49 | | 7.52 | 0.7208 | 55.10 |
| | | 15.960 | 91.508 | | | | | |

Analysis: The PAO was very unstable in the sesame oil, the total impurities increased to 9.95% after 4 h, and turbidity appeared at 20 h. In the soybean oil and the tea oil, the single impurities increased to 8.492% and 4.469% respectively after 48 h.

### 1.3 Investigation on stability of PAO in vegetable oil after addition of antioxidant

The stabilities after adding two different antioxidants (2,6-di-tert-butyl-4-methylphenol, vitamin E) to the tea oil and soybean oil including the PAO and after mixing the PAO with the vitamin E alone were investigated respectively.

**Table 6: Formulation composition of PAO after addition of antioxidant in experiment of stability**

| Lot number | F1 | F2 | F3 | F4 |
|---|---|---|---|---|
| Names of raw and auxiliary materials | Amount | | | |
| PAO | 10 mg | 10 mg | 10 mg | 10 mg |
| Soybean oil | 5 g | 5 g | - | - |
| Tea oil | - | - | 5 g | - |
| 2,6-di-tert-butyl-4-methylphenol (BHT) | 5.7 mg | - | 5.7 mg | - |
| Vitamin E (VE) | -- | 71.57 mg | - | 71.57 mg |

Methods: The components were weighed respectively according to the above formulation, and placed into a 20 ml vial. The mixture was stirred on a constant-temperature magnetic stirrer at room temperature for 30 min, and filtered through a 0.22 µm filter. The filtrate was collected, allowed to stand at room temperature, and diluted with isopropanol at 0 h, 1.5 h, 18 h, 24 h and 48 h respectively. The stability was investigated through HPLC detection.

Results:

**Table 7: Experimental results of stability of PAO in soybean oil after addition of BHT**

| Ingredients | Time | RT | % Measurements (area normalization method) | % Total impurities (area normalization method) | Appearance | Dilution fold | Concentration (mg/ml) | % API residue ratio |
|---|---|---|---|---|---|---|---|---|
| F1 (PAO+soybean oil+BHT) | 0 h | 6.436 | 0.321 | 0.32 | Clear oily liquid | 8.17 | 1.0816 | - |
| | | 16.343 | 99.679 | | | | | |
| | 1.5 h | 6.419 | 0.299 | 0.30 | | 7.26 | 1.0248 | 94.75 |
| | | 16.300 | 99.702 | | | | | |
| | 18 h | 6.265 | 0.980 | 0.98 | | 7.13 | 1.0383 | 96.00 |
| | | 15.927 | 99.020 | | | | | |
| | 24 h | 6.246 | 1.0698 | 1.07 | | 7.43 | 1.0326 | 95.47 |
| | | 15.892 | 98.9302 | | | | | |
| | 48 h | 6.188 | 1.366 | 1.37 | | 8.00 | 0.9592 | 88.68 |
| | | 15.819 | 98.634 | | | | | |
| | 4 d | 6.133 | 2.714 | 2.27 | | 7.16 | 0.8146 | 75.31 |
| | | 15.742 | 97.286 | | | | | |

**Table 8: Experimental results of stability of PAO in soybean oil after addition of VE**

| Ingredients | Time | RT | % Measurements (area normalization method) | % Total impurities (area normalization method) | Appearance | Dilution fold | Concentration (mg/ml) | % API residue ratio |
|---|---|---|---|---|---|---|---|---|
| F2 (PAO+soybean oil+VE) | 0 h | 6.416 | 1.002 | 1.00 | Clear oily liquid | 7.72 | 0.9619 | - |
| | | 16.335 | 98.998 | | | | | |
| | 1.5 h | 6.381 | 1.899 | 1.90 | | 7.65 | 0.8864 | 92.15 |
| | | 16.278 | 98.101 | | | | | |
| | 18 h | 6.208 | 68.605 | 68.60 | Turbid, with production of particles | 7.44 | 0.0911 | 9.47 |
| | | 15.933 | 31.396 | | | | | |
| | 24 h | 6.200 | 88.3043 | 88.30 | | 7.69 | 0.0569 | 5.92 |
| | | 15.886 | 11.6957 | | | | | |

**Table 9: Experimental results of stability of API in tea oil after addition of BHT**

| Ingredients | Time | RT | % Measurements (area normalization method) | % Total impurities (area normalization method) | Appearance | Dilution fold | Concentration (mg/ml) | % API residue ratio |
|---|---|---|---|---|---|---|---|---|
| F3 (PAO+tea oil+BHT) | 0 h | 6.173 | 0.363 | 0.36 | Clear oily liquid | 8.45 | 0.9485 | - |
| | | 15.931 | 99.637 | | | | | |
| | 1.5 h | 6.178 | 0.544 | 0.54 | | 7.84 | 0.9411 | 99.21 |
| | | 15.933 | 99.456 | | | | | |
| | 18 h | 6.120 | 1.153 | 1.15 | | 7.46 | 0.8750 | 92.25 |
| | | 15.848 | 98.847 | | | | | |
| | 24 h | 6.222 | 1.2143 | 1.21 | | 7.96 | 0.8967 | 94.54 |
| | | 15.901 | 98.7857 | | | | | |
| | 48 h | 6.178 | 1.203 | 1.20 | | 8.13 | 0.8513 | 89.75 |
| | | 15.823 | 98.797 | | | | | |
| | 4 d | 6.125 | 1.711 | 1.71 | | 7.90 | 0.8445 | 89.04 |
| | | 15.713 | 98.289 | | | | | |

**Table 10: Experimental results of stability of API in tea oil after addition of VE**

| Ingredients | Time | RT | % Measurements (area normalization method) | % Total impurities (area normalization method) | Appearance | Dilution fold | Concentration (mg/ml) | % API residue ratio |
|---|---|---|---|---|---|---|---|---|
| F4 (PAO+tea oil+VE) | 0 h | 6.186 | 0.436 | 0.44 | Clear oily liquid | 8.19 | 0.8530 | - |
| | | 15.938 | 99.565 | | | | | |
| | 1.5 h | 6.168 | 0.709 | 0.71 | | 7.74 | 0.8002 | 93.81 |
| | | 15.916 | 99.291 | | | | | |
| | 18 h | 6.125 | 2.334 | 2.33 | | 7.82 | 0.6844 | 80.23 |
| | | 15.844 | 97.666 | | | | | |
| | 24 h | 6.213 | 2.5903 | 2.59 | | 7.87 | 0.7055 | 82.70 |
| | | 15.898 | 97.4097 | | | | | |
| | 48 h | 6.171 | 3.464 | 3.46 | | 8.04 | 0.5860 | 68.70 |
| | | 15.825 | 96.536 | | | | | |
| | 4 d | 6.118 | 9.020 | 9.02 | | 7.89 | 0.4913 | 57.6 |
| | | 15.720 | 90.980 | | | | | |

Analysis: Compared with the results without the addition of antioxidants, degradation of the PAO was improved after the antioxidants were added. The effect of adding BHT was better than that of adding VE, but from the perspective of the PAO content, there was still a significant reduction.

### Example 2: Solubility and stability of PAO in mono-/di-glycerides of octanoic/decanoic acid (MCM) and medium-chain triglycerides (MCT)

### 2.1 Investigation on stability of API in mono-/di-glycerides of octanoic/decanoic acid

Formulation:

**Table 11: Formulation of PAO in mono-/di-glycerides of octanoic/decanoic acid in experiment of stability**

| Names of raw and auxiliary materials | Weight |
|---|---|
| PAO | 10 mg |
| Mono-/di-glycerides of octanoic/decanoic acid (MCM) | 5 g |
| BHT | 8 mg |

Methods:
1. The raw and auxiliary materials were weighed respectively according to the above formulation, and placed into a 20 ml vial. The mixture was stirred magnetically at room temperature for 30 min.
2. After stirring, the mixture was filtered through a 0.22 µm nylon millipore filter with a diameter of 25 mm. The filtrate was detected by HPLC for the content and related substances.
3. The sample was placed at room temperature, away from light, and sampled and detected on day 2, day 5 and day 11.

Results:

**Table 12: Experimental results of 11-day stability of PAO in mono-/diglycerides of octanoic/decanoic acid**

| Formulation composition | Time | RT | % Measurements | % Total impurities | Appearance | Concentration (mg/ml) |
|---|---|---|---|---|---|---|
| PAO+BHT +MCM | 0 d | 16.582 | 100.000 | 0.00 | Clear oily liquid | 100.1400 |
| | 2d | 16.582 | 100.000 | 0.00 | Clear oily liquid | 101.4020 |
| | 5 d | 15.622 | 100.000 | 0.00 | Clear oily liquid | 101.9920 |
| | 11 d | 6.546 | 0.247 | 0.25 | Clear oily liquid | 100.3560 |
| | | 15.812 | 99.753 | | | |

Analysis: The experimental results are shown in Table 12. No related substances were detected in the first 5 days, and the content remained substantially unchanged. By 11 d, the content still did not change significantly, but the related substance phenylarsonic acid increased to 0.25%.

### 2.2 Investigation on stability of PAO in medium-chain triglycerides

The stability of the PAO in medium-chain triglycerides (MCT) was investigated.

Formulation:

**Table 13: Formulation of PAO in medium-chain triglycerides in experiment of stability**

| Names of raw and auxiliary materials | Theoretical weight |
|---|---|
| PAO | 20 mg |
| MCT | 6.98 g |
| BHT | 8 mg |

Methods:
1. The raw and auxiliary materials were weighed respectively according to the above formulation, and placed into a 20 ml vial. The mixture was stirred at room temperature for 30 min.
2. After stirring, the mixture was filtered through a 0.22 µm nylon millipore filter with a diameter of 25 mm. The filtrate was detected by HPLC for the content and related substances.
3. The sample was placed at room temperature, away from light, and sampled and detected on day 5 and day 14.

Results:

**Table 14: Experimental results of 14-day stability of PAO in medium-chain triglycerides**

| Formulation composition | Time | RT | % Measurements | % Total impurities | Appearance | Weight (mg) | Volume (ml) | Concentration (mg/ml) | % Content |
|---|---|---|---|---|---|---|---|---|---|
| PAO+ BHT+ MCT | 0 d | 6.552 | 0.083 | 0.29 | Clear oily liquid | 502.2 | 5.00 | 100.4400 | 0.2347 |
| | | 15.591 | 99.709 | | | | | | |
| | | 31.121 | 0.208 | | | | | | |
| | 5 d | 6.576 | 0.196 | 0.40 | Clear oily liquid | 500.05 | 5.00 | 100.0100 | 0.2418 |
| | | 15.851 | 99.601 | | | | | | |
| | | 31.453 | 0.202 | | | | | | |
| | 14 d | 6.493 | 0.331 | 0.52 | Clear oily liquid | 508.17 | 5.00 | 101.6340 | 0.2222 |
| | | 15.676 | 99.481 | | | | | | |
| | | 31.358 | 0.189 | | | | | | |

Analysis: From the above experimental results, it can be known that the total related substances of the sample were 0.29% on day 0, increased to 0.40% on 5 d, and increased to 0.52% on day 14. The content of the phenylarsonic acid impurity (phenylarsonic acid) at retention time of 6.55 min was 0.083% on day 0, and increased to 0.33% after 14 days.

### 2.3 Investigation on stability of PAO in MCM and MCT solutions without addition of antioxidant BHT and influence of stirring time on dissolution

Formulation:

**Table 15: Formulation of PAO in MCT and MCM without addition of antioxidant BHT in the investigation on stability**

| Lot number | F13-180426 | F14-180426 |
|---|---|---|
| Names of raw and auxiliary materials | Theoretical weight | Theoretical weight |
| PAO | 40 mg | 20 mg |
| MCT | 20 g | - |
| MCM | - | 10 g |

Processes: F13-180426
1. The raw and auxiliary materials are weighed according to the above formulation, and placed into a 10 ml vial.
2. The mixture was stirred in a constant-temperature magnetic stirrer for 30 min. Approximately 10 g was taken, and filtered through a 0.22 µm millipore filter membrane with a diameter of 25 mm.
3. The remaining part was continuously stirred for 1 h, 2 h and 4 h, and then sampled. The samples were filtered through a 0.22 µm millipore filter membrane with a diameter of 25 mm.
4. The filtrates were detected by HPLC respectively.

F14-180426
1. The raw and auxiliary materials are weighed according to the above formulation, and placed into a 10 ml vial.
2. The mixture was stirred in a constant-temperature magnetic stirrer for 30 min.
3. The mixture was filtered through a 0.22 µm millipore filter membrane with a diameter of 25 mm. The filtrate was detected by HPLC.

Results:

**Table 16: The dissolution under different stirring times and stability results for 35 d room-temperature placing of F13 (PAO+MCT)**

| Formulation composition | Time | RT | % Measurements | % Total impurities | Appearance | Weight (mg) | Volume (ml) | Concentration (mg/ml) | % Content | % Content relative to 0 d |
|---|---|---|---|---|---|---|---|---|---|---|
| F13-180426 (PAO+ MCT) | 0 d (0.5 h) | 14.712 | 99.784 | 0.22 | Clear oily liquid | 757.95 | 5.00 | 151.5900 | 0.1944 | - |
| | | 30.670 | 0.216 | | | | | | | |
| | 0 d 1 h | 14.657 | 99.783 | 0.22 | Clear oily liquid | 753.13 | 5.00 | 150.6260 | 0.1963 | 100.98 |
| | | 30.632 | 0.218 | | | | | | | |
| | 2 h | 14.611 | 99.789 | 0.21 | Clear oily liquid | 761.95 | 5.00 | 152.3900 | 0.1999 | 102.83 |
| | | 30.626 | 0.211 | | | | | | | |
| | 4 h | 14.517 | 99.780 | 0.22 | Clear oily liquid | 758.61 | 5.00 | 151.7220 | 0.1998 | 102.78 |
| | | 30.571 | 0.220 | | | | | | | |
| | 6 d | 6.407 | 0.159 | 0.32 | Clear oily liquid | 748.96 | 5.00 | 149.7920 | 0.1930 | 99.28 |
| | | 15.485 | 99.682 | | | | | | | |
| | | 31.208 | 0.159 | | | | | | | |
| | 11 d | 6.471 | 0.121 | 0.30 | Clear oily liquid | 755.72 | 5.00 | 151.1440 | 0.1971 | 101.39 |
| | | 15.617 | 99.699 | | | | | | | |
| | | 31.302 | 0.180 | | | | | | | |
| | 15 d | 6.223 | 0.168 | 0.34 | Clear oily liquid | 761.07 | 5.00 | 152.2140 | 0.1947 | 100.15 |
| | | 15.080 | 99.665 | | | | | | | |
| | | 31.030 | 0.167 | | | | | | | |
| | 21 d | 5.948 | 0.140 | 0.30 | Clear oily liquid | 754.03 | 5.00 | 150.8060 | 0.1933 | 99.43 |
| | | 14.479 | 99.699 | | | | | | | |
| | | 30.632 | 0.161 | | | | | | | |
| | 27 d | 6.133 | 0.0816 | 0.29 | Clear oily liquid | 757.76 | 10 | 75.7760 | 0.2024 | 104.12 |
| | | 14.778 | 99.708 | | | | | | | |
| | | 30.741 | 0.138 | | | | | | | |
| | | 31.752 | 0.073 | | | | | | | |
| | 35 d | 6.652 | 0.2059 | 0.41 | Clear oily liquid | 761.96 | 10 | 76.1960 | 0.1923 | 98.92 |
| | | 15.976 | 99.593 | | | | | | | |
| | | 31.527 | 0.147 | | | | | | | |
| | | 32.476 | 0.054 | | | | | | | |

Analysis: From the above experimental results, it can be known that along with the extension of the stirring time, the content of the API in MCT substantially tended to be stable and overall approximated the theoretical content, namely 0.2% (w/w). The detection results on 0 d showed that no phenylarsonic acid impurities were produced, and only impurities of the active pharmaceutical ingredients themselves appeared near 31 min. With the continuation of room-temperature placing, the phenylarsonic acid at an amount of 0.159% began to appear from 6 d. The content of phenylarsonic acid at each subsequent time point fluctuated around the detection result on 6 d. It can be seen that after the API was placed in oil for a period of time, the phenylarsonic acid content tended to be stable.

**Table 17: Detection results of stability of F14 (PAO + MCM) for placement at room temperature for 35 d**

| Formulation composition | Time | RT | % Measurements | % Total impurities | Appearance | Weight (mg) | Volume (ml) | Concentration (mg/ml) | % Content | % Content relative to 0 d | RSD |
|---|---|---|---|---|---|---|---|---|---|---|---|
| F14-180426 (PAO+ MCM) | 0 d | 14.345 | 99.809 | 0.19 | Clear oily liquid | 752.58 | 5.00 | 150.5160 | 0.1930 | - | - |
| | | 30.465 | 0.191 | | | | | | | | |
| | 6 d | 6.35 | 0.116 | 0.26 | Clear oily liquid | 754.45 | 5.00 | 150.8900 | 0.1857 | 96.22 | - |
| | | 15.374 | 99.742 | | | | | | | | |
| | | 31.110 | 0.142 | | | | | | | | |
| | 11 d (taking upper layer) | 6.391 | 0.144 | 0.26 | Clear oily liquid | 759.10 | 5.00 | 151.8200 | 0.1924 | 99.69 | 0.86 |
| | | 15.432 | 99.741 | | | | | | | | |
| | | 31.171 | 0.115 | | | | | | | | |
| | 11 d (taking lower layer) | 6.395 | 0.157 | 0.27 | Clear oily liquid | 759.70 | 5.00 | 151.9400 | 0.1908 | 98.86 | |
| | | 15.401 | 99.726 | | | | | | | | |
| | | 31.164 | 0.118 | | | | | | | | |
| | 11 d (taking after being uniformly mixed) | 6.385 | 0.120 | 0.24 | Clear oily liquid | 754.27 | 5.00 | 150.8540 | 0.1941 | 100.57 | |
| | | 15.381 | 99.759 | | | | | | | | |
| | | 31.154 | 0.121 | | | | | | | | |
| | 15 d | 6.188 | 0.149 | 0.27 | Clear oily liquid | 759.08 | 5.00 | 151.8160 | 0.1861 | 96.42 | - |
| | | 15.019 | 99.735 | | | | | | | | |
| | | 30.967 | 0.116 | | | | | | | | |
| | 21 d | 5.906 | 0.144 | 0.46 | Clear oily liquid | 763.45 | 5.00 | 152.6900 | 0.1905 | 98.70 | - |
| | | 14.397 | 99.540 | | | | | | | | |
| | | 30.596 | 0.316 | | | | | | | | |
| | 27 d | 6.04 | 0.144 | 0.07 | Clear oily liquid | 756.53 | 10 | 75.6530 | 0.1968 | 101.97 | - |
| | | 14.617 | 99.931 | | | | | | | | |
| | 35 d | 6.595 | 0.284 | 0.28 | Clear oily liquid | 744.51 | 10 | 74.4510 | 0.1803 | 93.42 | |
| | | 15.829 | 99.716 | | | | | | | | |
| Pure PAO 0020182-029) | - | 14.144 | 99.699 | 0.30 | White powder | 20.63 | 20.00 | 1.0315 | 102.8 | - | - |
| | | 30.401 | 0.209 | | | | | | | | |
| | | 33.661 | 0.047 | | | | | | | | |
| | | 35.920 | 0.046 | | | | | | | | |

Analysis: For F14 taking MCM as a solvent, the overall trend of the stability was consistent with that of F13. Phenylarsonic acid began to appear from 6 d, was in a relatively stable state, and reached the maximum on 35 d.

### 2.4 Investigation on influence factors (high temperature, high humidity and light exposure) of F15 and F16

F15 and F16 were placed under high temperature (50°C), high humidity (92.5% RH) and light exposure (4,500 1x) respectively. Samples were taken on 5 d, 10 d and 30 d respectively to detect the content and related substances.

**Table 18: Formulation of F15 for influence factor investigation**

| Lot number | F15-180515 |
|---|---|
| Names of raw and auxiliary materials | Theoretical weight |
| PAO | 100 mg |
| MCT | 50 g |

**Table 19: Formulation of F16 for influence factor investigation**

| Lot number | F16-180515 |
|---|---|
| Names of raw and auxiliary materials | Theoretical weight |
| PAO | 100 mg |
| MCM | 50 g |

Methods:
F15-180515: The active pharmaceutical ingredients were passed through a 200-mesh screen. The raw and auxiliary materials were weighed, and placed into a vial. The mixture was magnetically stirred for 30 min, and filtered through a 0.22 µm millipore filter of 25 mm. The solution was taken triplicate, and placed under the conditions of high temperature of 50°C, high humidity of 92.5% RH and light exposure of 4,500 Lx respectively. Samples were taken on 5 d, 10 d and 30 d respectively to investigate the influence factors.

F16-180515: The MCM was heated in a water bath at 40°C for 3-5 min until the MCM was melted into a liquid, and the remaining steps are the same as those of F15 to perform investigation on the influence factors.

Results of the influence factors of F15-180515 are shown in Tables 20-23:

**Table 20: Analysis results of F15 in refrigerator (2-8°C) on 5 d, 10 d and 33 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **% Content relative to 0 d** |
|---|---|---|---|---|---|---|---|---|---|---|---|
| **F15-180515 (MCT+PAO) Low temperature (2-8°C)** | 0 d | 14.345 | 1.00 | 99.794 | 0.206 | Clear oily liquid | 759.00 | 10 | 75.9000 | 0.1544 | - |
| | | 30.584 | 2.13 | 0.206 | | | | | | | |
| | 5 d | 6.339 | 0.42 | 0.11 | 0.37 | | 761.78 | 10 | 76.1780 | 0.1635 | 105.89 |
| | | 15.217 | 1.00 | 99.629 | | | | | | | |
| | | 31.054 | 2.04 | 0.261 | | | | | | | |
| | 10 d | 6.596 | 0.42 | 0.275 | 0.47 | | 768.23 | 10 | 76.8230 | 0.1620 | 104.92 |
| | | 15.802 | 1.00 | 99.535 | | | | | | | |
| | | 31.479 | 1.99 | 0.190 | | | | | | | |
| | 33 d | 15.428 | 1.00 | 99.885 | 0.12 | | 757.02 | 10 | 75.7020 | 0.1545 | 100.06 |
| | | 31.264 | 2.03 | 0.115 | | | | | | | |
| Note: The 0 d results were measured on the day of sample formulation, and were the same data as other influence factors. The parts marked in red are for the impurity phenylarsonic acid. | | | | | | | | | | | |

**Table 21: Analysis results of F15 in high-humidity (92.5% RH) stability chamber on 5 d, 10 d and 32 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Relative content percentage** | |
|---|---|---|---|---|---|---|---|---|---|---|---|---|
| | | | | | | | | | | | Relative to 0 d | Relative to 2-8°C |
| **F15-180515 (MCT+ PAO) High humidity (92.5% RH)** | 5 d | 6.195 | 0.42 | 0.091 | 5.02 | Clear oily liquid | 750.85 | 10 | 75.0850 | 0.1630 | 105.57% | 99.69% |
| | | 14.875 | 1.00 | 94.576 | | | | | | | | |
| | | 30.795 | 2.07 | 0.252 | | | | | | | | |
| | | 31.857 | 2.14 | 0.404 | | | | | | | | |
| | | 32.838 | 2.21 | 4.676 | | | | | | | | |
| | 10 d | 6.453 | 0.42 | 0.1589 | 1.34 | | 748.56 | 10 | 74.8560 | 0.1626 | 105.31% | 100.37% |
| | | 15.467 | 1.00 | 98.834 | | | | | | | | |
| | | 31.218 | 2.02 | 0.258 | | | | | | | | |
| | | 32.190 | 2.08 | 0.131 | | | | | | | | |
| | | 33.180 | 2.15 | 0.918 | | | | | | | | |
| | 33 d | 6.522 | 0.41 | 0.5846 | 0.85 | | 746.06 | 10 | 74.6060 | 0.1547 | 100.19% | 100.13% |
| | | 15.721 | 1.00 | 99.148 | | | | | | | | |
| | | 31.376 | 2.00 | 0.146 | | | | | | | | |
| | | 32.339 | 2.06 | 0.122 | | | | | | | | |
| Note: The relative content percentages were respectively relative to the day of sample formulation and storage in the refrigerator for the same time period. The parts marked in red are for the impurity phenylarsonic acid. | | | | | | | | | | | | |

**Table 22: Analysis results of F15 in (50°C) stability chamber on 5 d, 10 d and 32 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Relative content percentage** | |
|---|---|---|---|---|---|---|---|---|---|---|---|---|
| | | | | | | | | | | | Relative to 0 d | Relative to 2-8°C |
| F15-180515 (MCT+PAO) High temperature (50°C) | 5 d | 6.287 | 0.42 | 0.1381 | 0.40 | Clear oily liquid | 750.64 | 10 | 75.0640 | 0.1620 | 104.92% | 99.08% |
| | | 15.130 | 1.00 | 99.603 | | | | | | | | |
| | | 30.986 | 2.05 | 0.259 | | | | | | | | |
| | 10 d | 6.52 | 0.42 | 0.3304 | 0.59 | | 757.43 | 10 | 75.7430 | 0.1623 | 105.17% | 100.19% |
| | | 15.698 | 1.00 | 99.410 | | | | | | | | |
| | | 31.383 | 2.00 | 0.209 | | | | | | | | |
| | | 32.357 | 2.06 | 0.051 | | | | | | | | |
| | 33 d | 6.631 | 0.42 | 0.4232 | 0.51 | | 747.50 | 10 | 74.7500 | 0.1572 | 101.81% | 101.75% |
| | | 15.917 | 1.00 | 99.491 | | | | | | | | |
| | | 31.543 | 1.98 | 0.086 | | | | | | | | |
| Note: The relative content percentages were respectively relative to the day of sample formulation and storage in the refrigerator for the same time period. The parts marked in red are for the impurity phenylarsonic acid. | | | | | | | | | | | | |

**Table 23: Analysis results of F15 in light exposure (4,500 1x) stability chamber on 5 d, 10 d and 32 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Relative content percentage** | |
|---|---|---|---|---|---|---|---|---|---|---|---|---|
| | | | | | | | | | | | **Relative** to 0 d | Relative to 2-8°C |
| **F15-180515 (MCT+ API) Light exposure (4,500 1x)** | 5 d | 6.2 | 0.41 | 3.4358 | 5.32 | Clear oily liquid | 764.03 | 10 | 76.4030 | 0.1395 | 90.35% | 85.32% |
| | | 15.003 | 1.00 | 94.680 | | | | | | | | |
| | | 21.766 | 1.45 | 1.825 | | | | | | | | |
| | | 30.859 | 2.06 | 0.059 | | | | | | | | |
| | 10 d | 6.43 | 0.41 | 4.8882 | 7.33 | | 757.87 | 10 | 75.7870 | 0.1240 | 80.31 % | 76.54% |
| | | 15.529 | 1.00 | 92.671 | | | | | | | | |
| | | 22.209 | 1.43 | 2.441 | | | | | | | | |
| | 33 d | 6.548 | 0.41 | 7.2746 | 25.45 | | 761.60 | 10 | 76.1600 | 0.0382 | 24.74% | 24.72% |
| | | 15.823 | 1.00 | 74.550 | | | | | | | | |
| | | 22.487 | 1.42 | 17.975 | | | | | | | | |
| | | 27.802 | 1.76 | 0.201 | | | | | | | | |
| Note: The relative content percentages were respectively relative to the day of sample formulation and storage in the refrigerator for the same time period. The parts marked in red are for the impurity phenylarsonic acid. | | | | | | | | | | | | |

Analysis: From the results, it can be known that the contents of F15 under low-temperature conditions on 5 d and 10 d were higher than those on 0 d (the day of sample formulation), and the impurity phenylarsonic acid was detected. The content result on 33 d was comparable to that on 0 d, and no phenylarsonic acid was detected.

The change trend of the API content under high-temperature and high-humidity conditions was consistent with that at low temperature. The phenylarsonic acid began to appear from 5 d. Under high-temperature and high-humidity conditions, the content of the phenylarsonic acid reached 0.42% and 0.58% respectively on 33 d. Under high-humidity conditions, new unknown impurities appeared after the retention time of 30 min. Under high-temperature conditions, similar impurities also appeared on 10 d, and the content was unstable.

Under light exposure conditions, the API degraded rapidly. On 33 d, the API content decreased to 24.72%, and the phenylarsonic acid content increased to 7.72%. In addition, a new unknown impurity (at the retention time of 22 min) began to appear from 5 d, and the impurity increased rapidly and increased to 17.98% on 33 d. At the same time, another new unknown impurity (at the retention time of 27.8 min) began to appear on 33 d.

Conclusions: After F15 was placed under various conditions for 33 d, the content of F15 changed to a certain extent under low-temperature, high-temperature and high-humidity conditions. The trends for the three conditions were the same, which first increased and then decreased. This change may be caused by the inaccurate content profiles of reference substances.

Related substances increased by different degrees under various conditions. The stability of the sample was poor under light exposure conditions. Along with the time extension of the placement, the API content decreased significantly, and the total impurity content increased significantly. The case at high temperature was comparable to that at high humidity, and the impurities increased slowly. For placing under high-temperature conditions for 5 d, only 0.138% of the phenylarsonic acid impurity appeared.

Results of the influence factors for F16 are shown in Tables 24-27:

**Table 24: Analysis results of F16 in refrigerator (2-8°C) stability chamber on 5 d, 10 d and 33 d**

| **Name** | **Tim e** | **RT** | **RR T** | **% Measuremen ts** | **% Total impuriti es** | **Appearan ce** | **Weig ht (mg)** | **Volum e (ml)** | **Concentrati on (mg/ml)** | **% Conte nt** | **Content percent a ge relative to 0 d** |
|---|---|---|---|---|---|---|---|---|---|---|---|
| **F16-180518-(MCM+PA O) Low temperatur e (2-8°C)** | 0 d | 14.12 1 | 1.00 | 99.794 | 0.206 | Clear oily liquid | 754.6 1 | 10 | 75.4610 | 0.1429 | - |
| | | 30.42 0 | 2.15 | 0.206 | | | | | | | |
| | 5 d | 14.64 5 | 1.00 | 100.000 | - | | 754.5 7 | 10 | 75.4570 | 0.1508 | 105.53% |
| | 10 d | 15.23 2 | 1.00 | 100.000 | - | | 752.6 4 | 10 | 75.2640 | 0.1492 | 104.41% |
| | 33 d | 6.269 | 0.41 | 0.526 | 0.53 | | 760.1 6 | 10 | 76.016 | 0.1426 | 99.79% |
| | | 15.16 8 | 1.00 | 99.474 | | | | | | | |
| Note: The relative content percentages were respectively relative to the day of sample formulation and storage in the refrigerator for the same time period. The parts marked in red are for the impurity phenylarsonic acid. | | | | | | | | | | | |

**Table 25: Analysis results of F16 in high-humidity (92.5% RH) stability chamber on 5 d, 10 d and 32 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Relative content percentage** | |
|---|---|---|---|---|---|---|---|---|---|---|---|---|
| | | | | | | | | | | | Relative to 0 d | Relative to 2-8°C |
| **F16-180518-(MCM+ PAO) High humidity (92.5% RH)** | 5 d | 14.611 | 1.00 | 100.000 | - | Clear oily liquid | 757.40 | 10 | 75.7400 | 0.1660 | 116.17% | 110.08% |
| | 10 d | 14.611 | 1.00 | 100.000 | - | | 747.76 | 10 | 74.7760 | 0.1649 | 115.40% | 110.52% |
| | 33 d | 6.335 | 0.41 | 0.789 | 0.79 | | 754.27 | 10 | 75.4270 | 0.1503 | 105.18% | 105.40% |
| | | 15.367 | 1.00 | 99.211 | | | | | | | | |
| Note: The relative content percentages were respectively relative to the day of sample formulation and storage in the refrigerator for the same time period. The parts marked in red are for the impurity phenylarsonic acid. | | | | | | | | | | | | |

**Table 26: Analysis results of F16 in high-temperature (50°C) stability chamber on 5 d, 10 d and 32 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Relative content percentage** | |
|---|---|---|---|---|---|---|---|---|---|---|---|---|
| | | | | | | | | | | | Relative to 0 d | Relative to 2-8°C |
| **F16-180518-(MCM+ PAO) High temperature (50°C)** | 5 d | 6.043 | 0.41 | 0.0654 | 0.07 | Clear oily liquid | 762.33 | 10 | 76.2330 | 0.1658 | 116.03% | 109.95% |
| | | 14.631 | 1.00 | 99.935 | | | | | | | | |
| | 10 d | 6.301 | 0.41 | 0.215 | 0.22 | | 761.63 | 10 | 76.1630 | 0.1647 | 115.26% | 110.39% |
| | | 15.212 | 1.00 | 99.785 | | | | | | | | |
| | 33 d | 6.35 | 0.41 | 1.0396 | 1.04 | | 757.74 | 10 | 75.7740 | 0.1504 | 105.25% | 105.47% |
| | | 15.397 | 1.00 | 98.960 | | | | | | | | |
| Note: The relative content percentages were respectively relative to the day of sample formulation and storage in the refrigerator for the same time period. The parts marked in red are for the impurity phenylarsonic acid. | | | | | | | | | | | | |

**Table 27: Analysis results of F16 in light exposure (4,500 LX) stability chamber on 5 d, 10 d and 32 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Relative content percentage** | |
|---|---|---|---|---|---|---|---|---|---|---|---|---|
| | | | | | | | | | | | Relative to 0 d | Relative to 2-8°C |
| **F16-180518-(MCM+ API) light exposure (4,500 LX)** | 5 d | 6.011 | 0.41 | 0.3294 | 0.33 | Clear oily liquid | 752.02 | 10 | 75.2020 | 0.1605 | 112.32% | 106.43% |
| | | 14.622 | 1.00 | 99.671 | | | | | | | | |
| | 10 d | 6.27 | 0.41 | 0.5011 | 0.50 | | 753.05 | 10 | 75.3050 | 0.1587 | 111.06% | 106.37% |
| | | 15.200 | 1.00 | 99.499 | | | | | | | | |
| | 33 d | 6.334 | 0.41 | 3.1528 | 3.15 | | 760.93 | 10 | 76.0930 | 0.1388 | 97.13% | 97.34% |
| | | 15.377 | 1.00 | 96.847 | | | | | | | | |
| Note: The 0 d results were measured on the day of sample formulation, and were the same data as other influence factors. The parts marked in red are for the impurity phenylarsonic acid. | | | | | | | | | | | | |

Analysis: In terms of related substances, the only impurity in F16 under various conditions was phenylarsonic acid. Because the MCM auxiliary material itself had a set of solvent peaks after the retention time of 30 min under this HPLC method, which overlapped with those of the impurities of the API in this area, the impurities of the API near here were not reflected in the table. After F16 was placed under low-temperature, high-humidity and high-temperature conditions for 33 d, the phenylarsonic acid increased more significantly than that in F15, reaching 0.53%, 0.79% and 1.04% respectively. Under light exposure conditions, the content of phenylarsonic acid in F16 was far lower than that in F15, and the main degradation impurity in F15 at the retention time of 22.48 min did not appear in F16.

In terms of the API content, the change trends under high-temperature and high-humidity conditions were the same. At each time point, the API content was higher than that under low-temperature conditions, and also higher than the 0 d detection result. On 0 d, because the formulated sample was placed in the refrigerator for several hours before being detected, the API content detected on 0 d was consistent with that at low temperature. Since MCM was solid at low temperature, it needed to be melted into a liquid before sampling during room-temperature detection. Therefore, the reason for the low content may be that the API was not completely re-dissolved in the MCM in the freezing and thawing process of the API in MCM solution, thus resulting in the low API content. Under light exposure conditions, the content of API relative to 0 d gradually decreased.

Conclusions: In general, F15 was less stable than F16 under light exposure conditions, but more stable than F16 under other influence factor conditions.

### 2.5 Placement stability forF15 and F16 (40°C/75% RH)and PAO in glyceryl monolinoleate (MAISINE CC) (40°C/75% RH and room temperature)

F15 and F16 were placed at 40°C/75% RH to investigate the stability. At the same time, a PAO in glyceryl monolinoleate solution was placed at 40°C/75% RH and at room-temperature, and samples were taken at different time points to investigate the stability.

**Table 28: Formulation of F15 and F16 and PAO in MAISINE CC solution for placement stability**

| **Lot number** | **F15-180601** | **F16-180601** | **F18-180601** |
|---|---|---|---|
| Names of auxiliary materials | Theoretical weight | Theoretical weight | Theoretical weight |
| PAO | 20 mg | 20 mg | 30 mg |
| MCT | 10 g | - | - |
| MCM | - | 10 g | - |
| MAISINE CC | - | - | 15 g |

Methods:
F15-180601: The active pharmaceutical ingredients were passed through an 80-mesh screen. The raw and auxiliary materials were weighed, and placed into a vial. The mixture was stirred on a magnetic stirrer at room temperature for 0.5 h, and filtered through a 0.22 µm nylon millipore filter. About 7 g of the filtrate was weighed, placed into a vial and then put into a 40°C/75% RH stability chamber. Samples were taken at different time points to investigate the stability. The remaining part of the filtrate was placed into a vial and then put into a 25°C/60°C stability chamber for later use.

F16-180601: The MCM was weighed, placed into a vial and then melted into a liquid in a water bath of 40°C, and the remaining steps are the same as those of F15-180601.

F18-180601: The formulation method was the same as that of F15-180601. The filtrate was divided into two parts, one part was placed in the laboratory, away from light, and the other part was placed in a 40°C/75% RH stability chamber. Samples were taken at different time points to investigate the stability.

Results:

**Table 29: Analysis results of content and related substances of F15 in (40°C/75% RH) stability chamber within 33 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Content percentage relative to 0 d** |
|---|---|---|---|---|---|---|---|---|---|---|---|
| F15-180601 (MCT+ PAO) | 0 d | 15.800 | 1.00 | 99.6146 | 0.39 | Clear oily liquid | 761.06 | 10 | 76.1060 | 0.1472 | - |
| | | 31.423 | 1.99 | 0.3247 | | | | | | | |
| | | 32.363 | 2.05 | 0.061 | | | | | | | |
| | 5 d (40°C/75% RH) | 15.355 | 0.49 | 99.838 | 0.16 | | 754.20 | 10 | 75.4200 | 0.1595 | 108.36 |
| | | 31.118 | 1.00 | 0.162 | | | | | | | |
| | 13d (40°C/75% RH) | 6.605 | 0.42 | 0.281 | 0.45 | | 746.92 | 10 | 74.6920 | 0.1515 | 102.92 |
| | | 15.841 | 1.00 | 99.555 | | | | | | | |
| | | 31.498 | 1.99 | 0.164 | | | | | | | |
| | **31 d** (40°C/75% RH) | 6.259 | 0.41 | 0.237 | 0.41 | | 750.18 | 10 | 75.0180 | 0.1532 | 104.08 |
| | | 15.184 | 1.00 | 99.586 | | | | | | | |
| | | 30.581 | 2.01 | 0.177 | | | | | | | |
| Note: The parts marked in red are for the impurity phenylarsonic acid. | | | | | | | | | | | |

**Table 30: Analysis results of content and related substances of F16 in (40°C/75% RH) stability chamber within 33 d**

| **Name** | **Time** | **RT** | **RRT** | **% Measurements** | **% Total impurities** | **Appearance** | **Weight (mg)** | **Volume (ml)** | **Concentration (mg/ml)** | **% Content** | **Content percentage relative to 0 d** |
|---|---|---|---|---|---|---|---|---|---|---|---|
| F16-180601 (MCM +PAO) | 0 d | 15.621 | 1.00 | 100.000 | - | Clear oily liquid | 746.99 | 10 | 74.699 | 0.1331 | - |
| | 5 d (40°C/75% RH) | 15.161 | 1.00 | 100.000 | - | | 763.13 | 10 | 76.3130 | 0.1342 | 100.83% |
| | 13 d (40°C/75% RH) | 6.517 | 0.42 | 0.458 | 0.46 | | 753.17 | 10 | 75.3170 | 0.1339 | 100.60% |
| | | 15.648 | 1.00 | 99.542 | | | | | | | |
| | 31 d (40°C/75% RH) | 6.15 | 0.41 | 0.298 | 0.30 | | 754.98 | 10 | 75.4980 | 0.1365 | 102.55% |
| | | 15.052 | 1.00 | 99.702 | | | | | | | |
| Note: The parts marked in red are for the impurity phenylarsonic acid. | | | | | | | | | | | |

**Table 31: Analysis results of content and related substances of F16 in (40°C/75% RH) stability chamber and under room-temperature conditions within 5 d**

| Name | Time | RT | RRT | % Measurements | % Total impurities | Appearance | Weight (mg) | Volume (ml) | Concentration (mg/ml) | % Content |
|---|---|---|---|---|---|---|---|---|---|---|
| F18-180601 (Maisine +PAO) | 0 d | 6.55 | 0.42 | 4.5601 | 4.56 | Yellow clear oily liquid | 748.54 | 10 | 74.8540 | 0.0619 |
| | | 15.753 | 1.00 | 95.440 | | | | | | |
| | 5 d (room-temperature laboratory) | 6.273 | 1.00 | 100.000 | - | | 771.89 | 10 | 77.1890 | - |
| | 5 d (40°C/75% RH) | 6.267 | 1.00 | 100.000 | - | | 753.72 | 10 | 75.3720 | - |
| Note: The parts marked in red are for the impurity phenylarsonic acid. | | | | | | | | | | |

Analysis: For F15, no phenylarsonic acid was detected on 0 d, but an unknown related substance appeared at the retention time of 32 min. It was later confirmed that this impurity was an external pollution impurity. In addition, the phenylarsonic acid impurity began to appear from 13 d, reaching 0.28%; and it decreased slightly after 31 d. The content of PAO was generally higher than that on 0 d and reached the maximum on 5 d, and the content relative to 0 d reached 108.36%.

For F16, the content substantially tended to be stable. The change trends of the related substances in F16 were consistent with those exhibited in F15. The phenylarsonic acid impurity began to appear from 13 d, reaching 0.46%; and it also decreased slightly on 31 d. At the same time point, the phenylarsonic acid content was higher than that of F15.

For F18 with glyceryl monolinoleate as the matrix, the PAO was extremely unstable therein, and 4.56% phenylarsonic acid was detected on the day of formulation. The PAO was completely degraded both at room temperature and in a 40°C/75% RH stability chamber on 5 d.

Conclusions: Based on the comprehensive comparison stability results of F15 and F16 under 40°C/75% RH conditions within 31 d, the stability of F15 was slightly higher than that of F16. In addition, compared with previous stability results at room temperature, the stability of F15 under accelerated conditions (40°C/75% RH) was comparable to that at room temperature, while the placement stability of F16 under accelerated conditions (40°C/75% RH) was slightly lower than that at room temperature.

### 2.6 Placement stability of PAO sample (25°C/60% RH and 2-8°C)

PAO samples (PAO in MCT solutions, having a concentration of 1.5 mg/ml) were stored under 25°C/60% RH (accelerated) and 2-8°C (long-term) conditions for 6 months respectively. The HPLC test results of the stability are shown in Table 32 and Table 33. The HPLC analysis method and parameters are substantially the same as those in Table 2, except that the mobile phase A is changed from 0.05% TFA aqueous solution to 0.05% H₃PO₄ aqueous solution.

**Table 32: Accelerated stability test results of PAO sample stored under 25°C/60% RH conditions for 6 months**

| Test item | Method | N/A | Time (month) | | | | |
|---|---|---|---|---|---|---|---|
| | | | 0 | 1 | 2 | 3 | 6 |
| Properties | GAM-GP-QC-012 | | Colorless clear oily liquid | Colorless clear oily liquid | Colorless clear oily liquid | Colorless clear oily liquid | Colorless clear oily liquid |
| Clarity | ChP <0902> | | Compliant | Compliant | Compliant | Compliant | Compliant |
| Related substances | AM-DCG025-01 | Names of impurities | % Impurities | | | | |
| | | RRT0.39 (0.38-0.40) | ND | ND | ND | ND | ND |
| | | RRT0.45 (0.44-0.46):PA | 0.25 | 022 | 0.24 | 0.27 | 0.11 |
| | | RRT1.08 (1.09) | ND | ND | ND | ND | N/A |
| | | RRT1.25 (1.24) | ND | ND | ND | ND | ND |
| | | RRT1.37 (1.39) | ND | N/A | N/A | N/A | N/A |
| | | RRT1.39 | ND | N/A | N/A | N/A | N/A |
| | | RRT1.41 (1.39-1.42) | ND | ND | ND | ND | ND |
| | | RRT1.48 (1.46-1.49) | 0.12 | 0.13 | 0.13 | 0.13 | 0.14 |
| | | RRT1.67 (1.64-1.68) | 0.20 | 0.20 | 0.20 | 0.19 | 0.19 |
| | | RRT1.79 (1.76-1.80) | <LOQ (0.03) | <LOQ (0.03) | <LOQ (0.03) | <LOQ (0.03) | ND |
| | | RRT2.06 (2.02-2.07) | ND | ND | ND | <LOQ (0.03) | ND |
| | | % Total impurities | 0.57 | 0.54 | 0.57 | 0.60 | 0.44 |
| Content | AM-DCG025-01 | N/A | 100.1% | 99.5% | 100.0% | 98.6% | 97.7% |
| Microbial limit | AM-PI01-04 | N/A | Total aerobic bacteria: <10² CFU/mL Molds and yeasts: <50 CFU/mL Escherichia coli: Not detected per 1 mL | | | | |

| | | | | | | | |
|---|---|---|---|---|---|---|---|
| ND: Below the detection limit (0.03%); N/A: not applicable | | | | | | | |

**Table 33: Long-term stability test results of PAO sample stored under 2-8°C conditions for 6 months**

| Test item | Method | N/A | Time (month) | | |
|---|---|---|---|---|---|
| | | | 0 | 3 | 6 |
| Properties | GAM-GP-QC-012 | | Colorless clear oily liquid | Colorless clear oily liquid | Colorless clear oily liquid |
| Clarity | ChP <0902> | | Compliant | Compliant | Compliant |
| Related substances | AM-DCG025-01 | Names of impurities | % Impurities | | |
| | | RRT0.39 (0.38-0.40) | ND | ND | ND |
| | | RRT0.45 (0.44-0.46):PA | 0.25 | 0.15 | 0.10 |
| | | RRT1.08 (1.09) | ND | ND | N/A |
| | | RRT1.25 (1.24) | ND | ND | ND |
| | | RRT1.37 (1.39) | ND | N/A | N/A |
| | | RRT1.39 | ND | N/A | N/A |
| | | RRT1.41 (1.39-1.42) | ND | ND | ND |
| | | RRT1.48 (1.46-1.49) | 0.12 | 0.14 | 0.13 |
| | | RRT1.67 (1.64-1.68) | 0.20 | 0.20 | 0.20 |
| | | RRT1.79 (1.76-1.80) | <LOQ (0.03) | <LOQ (0.03) | ND |
| | | RRT2.06 (2.02-2.07) | ND | <LOQ (0.03) | ND |
| | | % Total impurities | 0.57 | 0.48 | 0.44 |
| Content | AM-DCG025-01 | N/A | 100.1% | 99.1% | 98.2% |
| Microbial limit | AM-PI01-04 | N/A | | | Total aerobic bacteria: <10² CFU/mL Molds and yeasts: <50 CFU/mL Escherichia coli: Not detected per 1 mL |

| | | | | | |
|---|---|---|---|---|---|
| ND: Below the detection limit (0.03%); N/A: not applicable | | | | | |

### Example 3: Screening formulation conditions of PAO in MCT solution

Experiments on the influence factors of F15 showed that API was relatively stable in MCT under high-temperature (50°C) conditions. Therefore, it is considered to promote the dissolution of PAO by heating.

**Table 34: Formulation for dissolution of API by heating**

| **Lot number** | **F15-180929** |
|---|---|
| Names of auxiliary materials | Theoretical weight |
| PAO | 20 mg |
| MCT | 10 g |

Methods: The temperature of a constant-temperature magnetic stirrer was preset at 50°C. After the temperature reached 50°C, the raw and auxiliary materials were weighed and placed into a 25 ml round-bottom flask. The mixture was stirred at a set speed of 800 rpm in the dark, and phenomena were observed at different time points. After the mixture became clear, a sample was taken and filtered through a nylon millipore filter membrane with a pore size of 0.22 µm. The content and related substances were detected by HPLC.

Status of sample:

**Table 35: Phenomena of PAO at different time points during dissolution by heating**

| **Time point** | **Phenomena** |
|---|---|
| Very beginning of stirring | PAO was suspended in MCT, and the system was turbid, with a large number of obvious big particles. |
| Stirring for 15 min | Except for a small number of visible big particles, the system appeared to be clear. |
| Stirring for 30 min | No visible undissolved substances were found, and the system appeared to be clear and transparent. |
| Stirring for 1 h | Phenomena were the same as those at the time point of stirring for 30 min. |
| Stirring for 2 h | Phenomena were the same as those at the time point of stirring for 30 min. |
| Stirring for 4 h | Phenomena were the same as those at the time point of stirring for 30 min. |

Results:

**Table 36: HPLC detection results of F15 at different time points during dissolution by heating**

| **Name** | **Sampling time point** | **RT** | **% Measurements** | **% Total impurities** | **Appearance** | **% Content** | **% RSD** |
|---|---|---|---|---|---|---|---|
| F15-180929 (PAO+MCT) | 0.5 h | 14.945 | 99.4157 | 0.58 | Clear oily liquid | 0.2005 | 0.53 |
| | | 30.517 | 0.3651 | | | | |
| | | 31.446 | 0.0462 | | | | |
| | | 35.520 | 0.1731 | | | | |
| | 1 h | 14.916 | 99.2742 | 0.73 | | 0.2017 | |
| | | 28.050 | 0.1021 | | | | |
| | | 30.488 | 0.3745 | | | | |
| | | 31.429 | 0.0565 | | | | |
| | | 35.502 | 0.1927 | | | | |
| | 2 h | 14.885 | 99.3120 | 0.69 | | 0.1991 | |
| | | 27.997 | 0.0946 | | | | |
| | | 30.455 | 0.3791 | | | | |
| | | 31.385 | 0.0539 | | | | |
| | | 35.482 | 0.1603 | | | | |
| | 4 h | 14.842 | 99.3127 | 0.69 | | 0.2006 | |
| | | 27.965 | 0.0894 | | | | |
| | | 30.413 | 0.3891 | | | | |
| | | 31.359 | 0.0515 | | | | |
| | | 35.459 | 0.1573 | | | | |

Analysis: The results are shown in Table 36. After the sample is stirred to become clear (0.5 h-4 h), the content reached the theoretical concentration (0.2%), and no phenylarsonic acid was generated. At the same time, an impurity at the retention time of 28 min was newly produced after 1 h. This impurity resulted from the active pharmaceutical ingredients. Therefore, stirring and heating for 0.5 h was the optimal formulation condition.

### Example 4: In vitro release experiments of PAO

Four formulations were prepared, and in vitro experiments were carried out to simulate the release of the formulations in the stomach. Since PAO had a higher affinity with proteins, a 0.1N HCl solution was temporarily used instead of artificial gastric juice. The unified formulation concentration was 2 mg/g. The release of the formulation was investigated through a dissolution instrument.

**Table 37: Dissolution method for in vitro release experiments**

| Release medium/volume | Speed | Sampling points | Temperature | Method |
|---|---|---|---|---|
| 0.1N hydrochloric acid/200 ml | 100 rpm | 15 min, 30 min, 45 min, 60 min and 120 min | 37±0.5°C | Paddle dissolution method |

### 4.1 Investigation on dissolution and stability of PAO in 0.1N HCl

20 mg of PAO was weighed, and dissolution experiments were carried out according to the above dissolution method (repeated once for the same sample). Samples were taken at different time points with a sampling volume of 3 ml, and no solution was supplemented after sampling. Each sample was subjected to 0.22 µm filtration. HPLC detection was performed on the filtrate to investigate the dissolution rate and stability. The dissolution solution at 2 h was continuously injected within 24 h to investigate the stability.

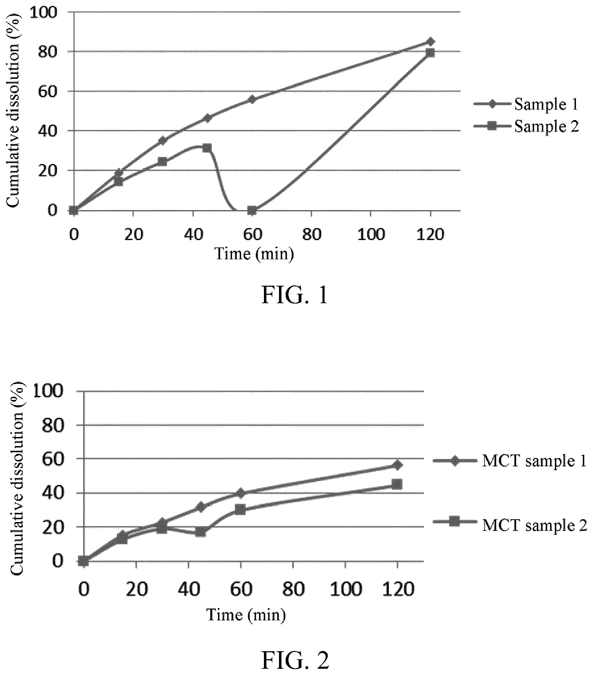

The results are shown in FIG. 1 and Tables 38-41:

**Table 38: Investigation on dissolution rate of PAO through dissolution method**

| Sample information | PAO sample 1 | PAO sample 2 |
|---|---|---|
| Time (min) | Cumulative release (%) | |
| 0 | 0 | 0 |
| 15 | 18.77 | 14.17 |
| 30 | 35.13 | 24.33 |
| 45 | 46.68 | 30.92 |
| 60 | 55.83 | 0.00 (data missing due to an HPLC problem) |
| 120 | 85.04 | 79.29 |

**Table 39: Related substances of dissolution sample of PAO sample 1**

| Time | RT | % Area | % Total impurities |
|---|---|---|---|
| 15 min | 5.539 | 0.3937 | 1.80 |
| | 7.613 | 1.0269 | |
| | 15.841 | 98.1977 | |
| | 20.882 | 0.3817 | |
| 30 min | 5.541 | 0.6226 | 6.17 |
| | 7.632 | 3.1118 | |
| | 15.864 | 93.8296 | |
| | 18.540 | 0.3762 | |
| | 20.882 | 2.0599 | |
| 45min | 5.528 | 0.4429 | 7.34 |
| | 7.606 | 4.3722 | |
| | 15.830 | 92.6648 | |
| | 18.465 | 0.5120 | |
| | 20.141 | 0.3500 | |
| | 20.89 | 1.6580 | |
| 1 h | 5.517 | 0.4258 | 7.43 |
| | 7.571 | 4.8278 | |
| | 15.813 | 92.5727 | |
| | 18.443 | 0.3926 | |
| | 20.145 | 0.3062 | |
| | 20.885 | 1.4748 | |
| 2 h | 5.503 | 0.5 | 5.44 |
| | 7.566 | 2.8557 | |
| | 15.805 | 94.5582 | |
| | 16.786 | 0.0593 | |
| | 18.439 | 0.2538 | |
| | 20.877 | 1.7730 | |

**Table 40: Related substances of dissolution sample of PAO sample 2**

| Time | RT | % Area | % Total impurities |
|---|---|---|---|
| 15 min | 5.54 | 0.7842 | 7.01 |
| | 7.615 | 3.8508 | |
| | 15.855 | 92.9863 | |
| | 18.525 | 0.5621 | |
| | 20.966 | 1.8166 | |
| 30 min | 5.528 | 0.69 | 6.03 |
| | 7.615 | 3.8168 | |
| | 15.870 | 93.9682 | |
| | 18.547 | 0.2166 | |
| | 20.974 | 1.3085 | |
| 45 min | 5.512 | 0.6174 | 7.13 |
| | 7.583 | 3.5237 | |
| | 15.819 | 92.6648 | |
| | 16.797 | 0.0880 | |
| | 18.458 | 0.6202 | |
| | 20.887 | 2.2803 | |
| 1 h | 5.498 | 0.9555 | 6.39 |
| | 7.559 | 2.9402 | |
| | 15.799 | 93.6145 | |
| | 16.774 | 0.0634 | |
| | 18.433 | 0.4308 | |
| | 20.122 | 0.0413 | |
| | 20.876 | 1.9543 | |
| 2 h | 5.514 | 0.5835 | 8.26 |
| | 7.586 | 4.8660 | |
| | 15.817 | 91.7416 | |
| | 16.806 | 0.1048 | |
| | 17.560 | 0.0743 | |
| | 18.450 | 0.3442 | |
| | 20.885 | 2.2857 | |

**Table 41: 24 h (liquid injection plate) stability detection results of dissolution sample at 2 h of PAO sample 1**

| Time | RT | % Area | % Total impurities |
|---|---|---|---|
| 0 h | 5.514 | 0.5835 | 8.26 |
| | 7.586 | 4.8660 | |
| | 15.817 | 91.7416 | |
| | 16.806 | 0.1048 | |
| | 17.560 | 0.0743 | |
| | 18.450 | 0.3442 | |
| | 20.885 | 2.2857 | |
| 2 h | 5.514 | 0.3373 | 7.89 |
| | 7.590 | 4.9222 | |
| | 15.820 | 92.1117 | |
| | 16.789 | 0.1023 | |
| | 18.446 | 0.2885 | |
| | 20.878 | 2.2380 | |
| 4 h | 5.528 | 0.3084 | 7.98 |
| | 7.602 | 4.8785 | |
| | 15.832 | 92.0247 | |
| | 16.812 | 0.1277 | |
| | 17.580 | 0.0406 | |
| | 18.463 | 0.3277 | |
| | 20.892 | 2.2924 | |
| 8 h | 5.524 | 0.3731 | 8.23 |
| | 7.603 | 4.9485 | |
| | 15.843 | 91.7686 | |
| | 16.814 | 0.1095 | |
| | 17.571 | 0.1033 | |
| | 18.469 | 0.4172 | |
| | 20.891 | 2.2798 | |
| 12 h | 5.524 | 0.3531 | 7.95 |
| | 7.595 | 4.9000 | |
| | 15.848 | 92.0488 | |
| | 16.837 | 0.1339 | |
| | 18.528 | 0.2220 | |
| | 20.963 | 2.3423 | |
| 24 h | 5.555 | 0.2967 | 7.84 |
| | 7.664 | 4.8424 | |
| | 15.886 | 92.1565 | |
| | 16.852 | 0.1088 | |
| | 18.559 | 0.3403 | |
| | 20.961 | 2.2552 | |

Analysis: In the experiment, from the time when PAO powder was added to a dissolution medium to the end of the dissolution, the undissolved PAO remained floating on the surface of the dissolution medium, and no suspension was found in the medium, showing poor wettability; and there were relatively fewer floating substances after dissolution experiment. In view of the profile, the API was in a dissolved state all the way, and the profile did not show slowing down significantly. In the dissolution process, a large number of impurities were produced, and irregular changes occurred to the impurities. This may be due to the low solubility of phenylarsonic acid, the main degradation impurity of API, in acid, the dissolution time and state or the like. The related substances substantially tended to be stable after 24-hour continuous injection of the dissolution sample at 2 h. Light exposure occurred in the dissolution process, but 24 h stability was measured in the injection plate, which was the stability against light exposure. Therefore, it can be determined that the API was stable in 0.1N hydrochloric acid. However, strict protection from light was required in the dissolution process.

### 4.2 Simulated release of PAO in MCT solution and glyceryl behenate solid dispersion

Sample preparation methods:
F1: The same as F15-180929.

F2: 15 g of glyceryl behenate was heated to 85°C until the glyceryl behenate was melted into a liquid, and then 30 mg of PAO was added and dissolved therein. The resulting product was cooled to room temperature, and granulated by sieving through a 30-mesh screen. Thus, a behenate solid dispersion containing 2 mg/g PAO was prepared.

Experimental methods for simulating release: 5 g of MCT solution and 5 g of the glycerol behenate solid dispersion (2 mg/g) were taken separately (repeated once for each sample). Release experiments were carried out according to the above method with a sampling volume of 3 ml, and no solution was supplemented after sampling. The sample was filtered through a 0.22 µm millipore filter membrane. The filtrate was investigated for the release by HPLC. After the completion of the dissolution experiment, the floating MCT oil and glyceryl behenate solid dispersion powder were collected, diluted and dissolved, and detected by HPLC to investigate related substances.

The results are shown in FIGs. 2-3 and Tables 42-45:

**Table 42: Cumulative release of MCT solution (F1)**

| Sample information | MCT sample 1 | MCT sample 2 |
|---|---|---|
| Time (min) | Cumulative release (%) | |
| 0 | 0 | 0 |
| 15 | 15.24 | 12.81 |
| 30 | 22.78 | 19.04 |
| 45 | 31.72 | 17.24 |
| 60 | 39.58 | 29.96 |
| 120 | 56.29 | 44.52 |

**Table 43: Cumulative release of glyceryl behenate (F2)**

| Sample information | Glyceryl behenate solid dispersion 1 | Glyceryl behenate solid dispersion 2 |
|---|---|---|
| Time (min) | Cumulative release (%) | |
| 0 | 0 | 0 |
| 15 | 1.38 | 1.28 |
| 30 | 2.99 | 0.00 |
| 45 | 4.76 | 2.05 |
| 60 | 7.89 | 5.78 |
| 120 | 10.97 | 1.70 |

Table 44: Related substances of floating MCT oil after the dissolution experiment of F1

| Name | RT | % Area | % Total impurities |
|---|---|---|---|
| Sample 1 | 9.271 | 1.5237 | 4.13 |
| | 17.780 | 95.8681 | |
| | 19.324 | 0.1311 | |
| | 24.658 | 0.0494 | |
| | 26.758 | 0.1878 | |
| | 28.61 | 0.141 | |
| | 30.37 | 0.5875 | |
| | 32.566 | 0.156 | |
| | 34.27 | 0.0658 | |
| | 36.139 | 0.1153 | |
| | 39.812 | 0.7797 | |
| | 40.438 | 0.3946 | |

**Table 45: Related substances of floating glyceryl behenate after the dissolution experiment of F2**

| Name | RT | % Area | % Total impurities |
|---|---|---|---|
| Sample 1 | 5.29 | 0.023 | 3.09 |
| | 9.247 | 1.5957 | |
| | 17.889 | 96.9111 | |
| | 19.519 | 0.0666 | |
| | 24.81 | 0.085 | |
| | 27.261 | 0.1406 | |
| | 27.788 | 0.2861 | |
| | 29.181 | 0.0558 | |
| | 30.984 | 0.4433 | |
| | 33.238 | 0.07 | |
| | 33.985 | 0.0414 | |
| | 37.368 | 0.1065 | |
| | 41.045 | 0.175 | |
| Sample 2 | 5.258 | 0.0221 | 2.79 |
| | 9.115 | 1.2046 | |
| | 17.773 | 97.2062 | |
| | 19.426 | 0.0888 | |
| | 24.705 | 0.1154 | |
| | 27.152 | 0.1269 | |
| | 27.662 | 0.4231 | |
| | 30.841 | 0.4272 | |
| | 33.086 | 0.0592 | |
| | 33.716 | 0.0857 | |
| | 37.076 | 0.0999 | |
| | 40.8 | 0.1407 | |

Analysis: The results are shown in the above tables. Compared with pure API, API was released more slowly from the MCT solution (F1), and did not reach a dissolution plateau at 2 h. This indicated that PAO was released from the MCT preparation in a sustained manner in the simulated gastric juice, which facilitated to reduce the topical irritation of PAO to the gastric mucosa. PAO was hardly released from the glyceryl behenate solid dispersion (F2). The solid dispersion was prepared from water insoluble glyceryl behenate, and particles were relatively fluffy. Accordingly, the sample powder was hardly wetted during the dissolution experiments but floated on the surface of the dissolution medium. Therefore, PAO was hardly released. Some impurities were newly produced for the sample after dissolution experiment. This may be caused by a fact such as light exposure or by the dissolution medium included in the dissolution residues.

### 4.3 In vitro simulated release experiments of PAO in MC suspension and PAO in MC suspension with Tween 80

Sample preparation methods: 10 mg of PAO and 5 g of methylcellulose (MC) aqueous solution (F3, with MC at a concentration of 2%, w/v) or an MC aqueous solution containing 0.1% (w/v) Tween 80 (F4, also with MC at a concentration of 2%, w/v) were weighed, and magnetically stirred for 30 min. Then, all the samples were added to the dissolution medium. In addition, samples of the same concentration were prepared respectively, and filtered through a 0.22 µm filter membrane. The content and related substances were detected by HPLC, and comprehensive analysis was performed according to the results.

Calculation method: The cumulative release was calculated by the initially input PAO which excludes PAO dissolved in the initial suspension. **Cumulative release** = [(API concentration at sampling point ^{∗} volume of release medium) + API concentration at previous sampling time point ^{∗} sampling volume at previous time point]/(input amount - dissolution concentration in suspension ^{∗} mass of suspension).

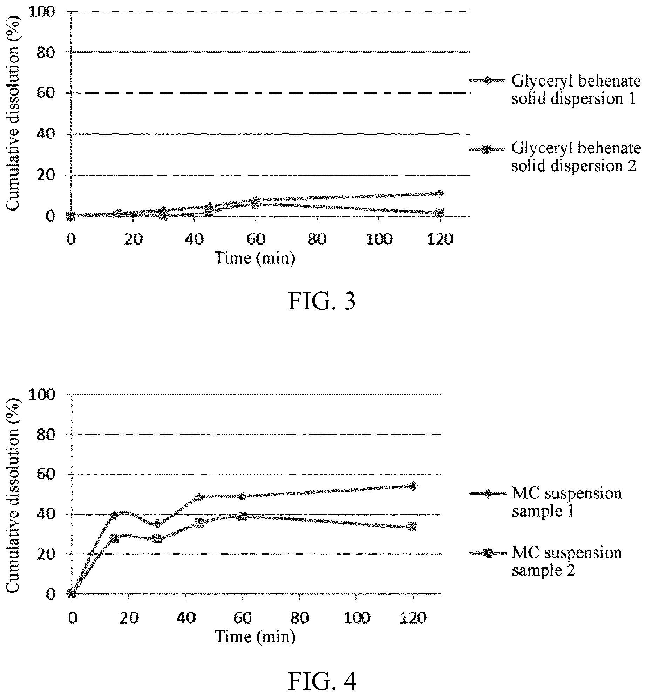

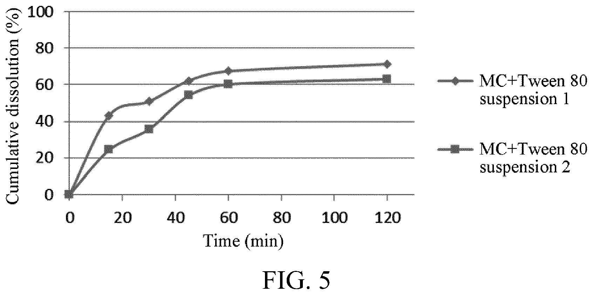

The results are shown in FIGs. 4-5 and Tables 46-49:

**Table 46: Cumulative release of MC suspension**

| Sample information | MC suspension 1 (F3) | MC suspension 2 (F4) |
|---|---|---|
| Time (min) | Cumulative release (%) | |
| 0 | 0 | 0 |
| 15 | 39.25 | 27.57 |
| 30 | 35.25 | 27.75 |
| 45 | 48.33 | 35.51 |
| 60 | 48.99 | 38.64 |
| 120 | 54.11 | 33.04 |

**Table 47: Cumulative release of MC+0.1 % Tween 80 suspension**

| Sample information | MC+ Tween 80 Suspension 1 (F4) | MC+ Tween 80 Suspension 2 (F5) |
|---|---|---|
| Time (min) | Cumulative release (%) | |
| 0 | 0 | 0 |
| 15 | 43.2 | 24.32 |
| 30 | 50.76 | 35.66 |
| 45 | 62.16 | 53.92 |
| 60 | 67.36175 | 60.16 |
| 120 | 71.32363 | 63.09175 |

**Table 48: Detection results of related substances for insoluble substances after the dissolution experiments of MC suspension and MC+Tween 80 suspension**

| Name | RT | RRT | Area | % Area | % Total impurities |
|---|---|---|---|---|---|
| F3 | 8.598 | 0.50 | 9.9086 | 0.759 | 3.07 |
| | 17.026 | 1.00 | 1265.4 | 96.9253 | |
| | 22.539 | 1.32 | 30.23259 | 2.3157 | |
| F4 | 8.046 | 0.49 | 9.33755 | 2.3907 | 4.31 |
| | 16.304 | 1.00 | 373.73837 | 95.6885 | |
| | 21.214 | 1.30 | 7.50208 | 1.9208 | |

**Table 49: Results of related substances for filtrate after sample formulation of MC suspension and MC+Tween 80 suspension**

| Name | RT | RRT | Area | % Area | % Total impurities |
|---|---|---|---|---|---|
| F3 | 8.003 | 0.49 | 9.45747 | 2.2153 | 2.22 |
| | 16.275 | 1.00 | 417.45660 | 97.7847 | |
| F4 | 8.046 | 0.49 | 9.33755 | 2.3907 | 4.31 |
| | 16.304 | 1.00 | 373.73837 | 95.6885 | |
| | 21.214 | 1.30 | 7.50208 | 1.9208 | |

Analysis: For the two methylcellulose suspensions, the release substantially reached a plateau at 1 h, and the cumulative release was close to that of the MCT solution. However, there were a small number of insoluble substances observed in the actual experiment process. In combination with the results of the related substances in the filtrate after sample formulation in Table 49, it can be inferred that the sample had poor stability in the two media and was highly degraded, thus resulting in a "pseudosustained release" condition in the simulated release experiments.

### Example 5: In vivo kinetic study of PAO in animals [0303] 5.1 In vivo kinetic study of oral MCT preparation of PAO in monkeys

Two groups of monkeys were selected, one male and one female in each group. PAO was orally administered to a first group (male 101 and female 102) by taking MCT as a vehicle at a dose of 0.3 mg/kg/day for 2 consecutive weeks. Blood was collected at 0.5, 1, 2, 4, 8, 12, 24, and 48 hours after administration on the last day to detect the concentration of the compound in the blood (whole blood, not plasma).

PAO was orally administered to a second group (male 301 and female 302) by similarly taking MCT as a vehicle at a dose of 0.3 mg/kg via single dosing. Blood was collected at 0.5, 1, 2, 4, 8, 12, 24, and 48 hours after administration to similarly detect the concentration of the compound in the whole blood. Afterwards, the administration was stopped for 5 days before PAO was orally administered by similarly taking MCT as a vehicle at a dose of 0.6 mg/kg via single dosing. Blood was collected at 0.5, 1, 2, 4, 8, 12, 24, and 48 hours after administration to detect the concentration of the compound in the whole blood.

**Table 50: Blood concentrations at different time**

| **Blood sample** | | | | | |
|---|---|---|---|---|---|
| **Grouping** | **Sampling time** | **Animal ID** | **Analyte** | **Concentration (ng/mL)** | **Average concentration (ng/mL)** |
| Compound PAO 0.3 mpk PO | 0.5 h | 101 102 | PI01 (PAO) | 80.9 83.4 | 82.2 |
| | | 301 302 | | 2.71 27.7 | 15.2 |
| | 1 h | 101 102 | | 93.6 83.0 | 88.3 |
| | | 301 302 | | 20.9 36.1 | 28.5 |
| | 2 h | 101 102 | | 109 130 | 120 |
| | | 301 302 | | 26.6 50.7 | 38.7 |
| | 4 h | 101 102 | | 172 141 | 157 |
| | | 301 302 | | 36.7 46.5 | 41.6 |
| | 8 h | 101 102 | | 26.9 63.2 | 45.1 |
| | | 301 302 | | 54.7 47.6 | 51.2 |
| | 12 h | 101 102 | | 27.1 57.7 | 42.4 |
| | | 301 302 | | 75.9 42.7 | 59.3 |
| | 24 h | 101 102 | | 89.3 110 | 99.7 |
| | | 301 302 | | 38.8 27.8 | 33.3 |
| | 48 h | 101 102 | | 99.8 95.2 | 97.5 |
| | | 301 302 | | 19.9 22.8 | 21.4 |
| Compound PAO, 0.6 mpk PO | 0.5 h | 301 302 | | 31.7 45.5 | 38.6 |
| | 1 h | 301 302 | | 126 144 | 135 |
| | 2 h | 301 302 | | 116 137 | 127 |
| | 4 h | 301 302 | | 125 141 | 133 |
| | 8 h | 301 302 | | 96.6 121 | 109 |
| | 12 h | 301 302 | | 71.6 99.4 | 85.5 |
| | 24 h | 301 302 | | 45.5 64.9 | 55.2 |
| | 48 h | 301 302 | | 32.9 51.1 | 42.0 |

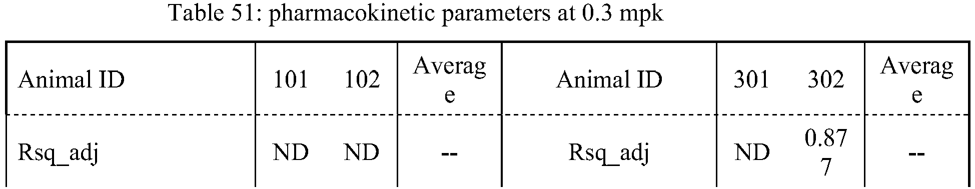

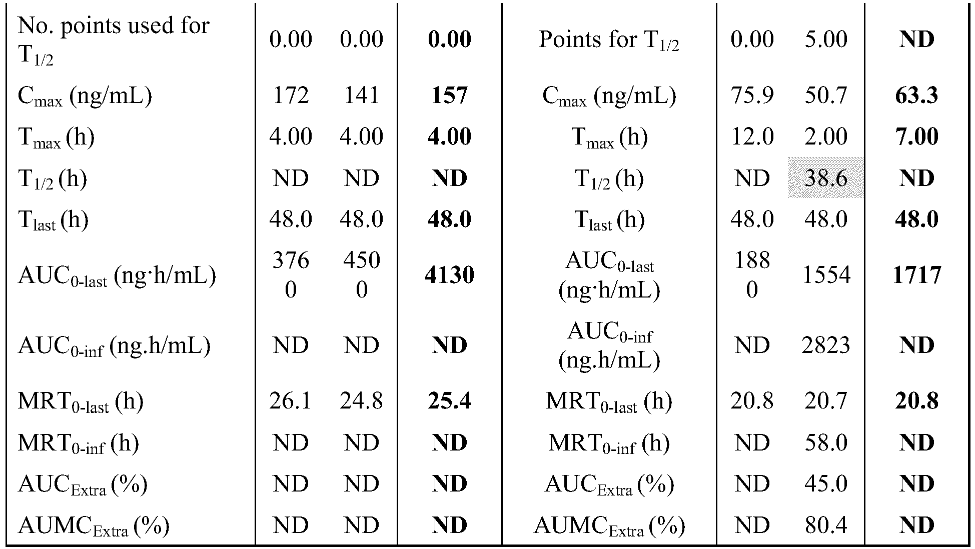

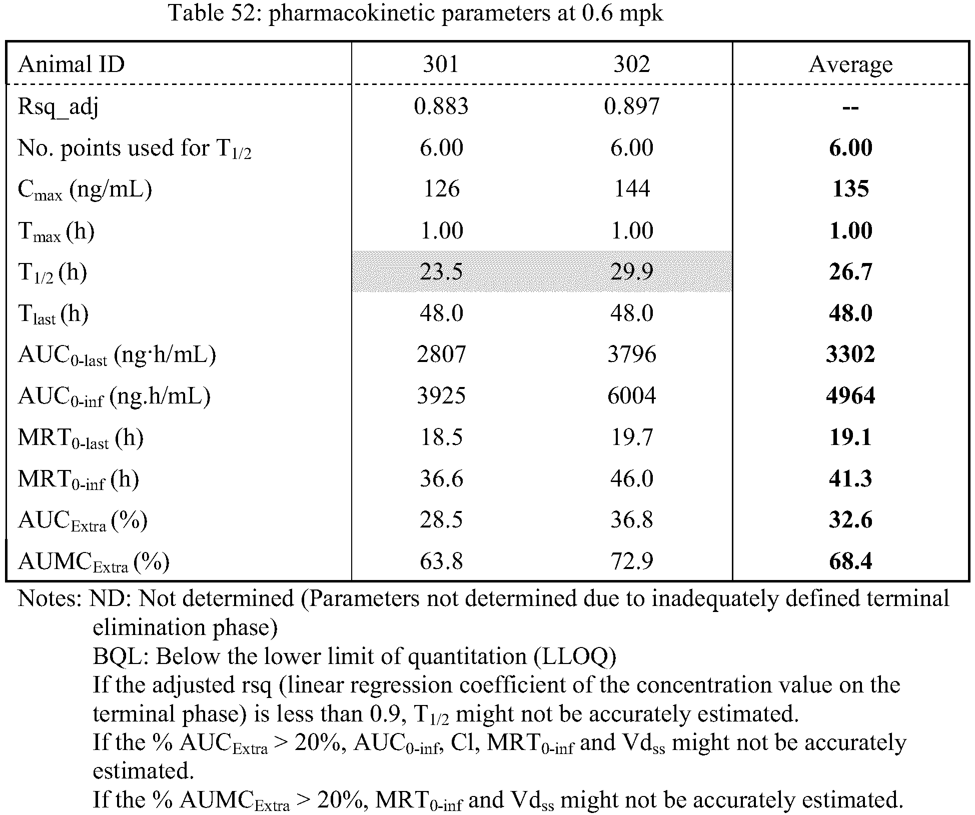

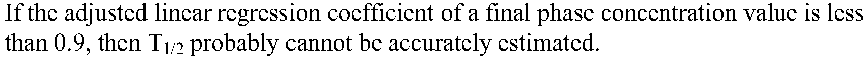

Analysis: After a single oral administration of the MCT preparation of PAO to the monkeys, PAO can be absorbed into the blood, and the blood concentration reached the maximum within 4 hours. PAO had a long half life in the blood, being about 26.7 hours, which indicated that a higher blood concentration can be maintained by orally administration of PAO once a day. In conclusion, this indicated that the MCT preparation of PAO can be used for oral delivery of PAO. After the MCT preparation of PAO was orally administered to the monkeys every day at a dose of 0.3 mg/kg/day for 2 consecutive weeks, the average exposure of PAO in the blood (AUC₀₋ₗₐₛₜ = 4130 ng·h/mL) was about 2.4 times as high as that of a single oral administration (AUC₀₋ₗₐₛₜ = 1717 ng·h/mL), indicating that repeated administration will cause medicament accumulation. It was suggested that the administration should be stopped for 1-2 days after the consecutive daily oral administration of the medicament for 2 weeks or within 2 weeks.

### 5.2 In vivo kinetic study of oral sesame oil preparation of PAO and intravenous PAO in monkeys

Fatty acids in MCT are mainly medium-chain saturated fatty acids, while fatty acids in sesame oil are mainly long-chain unsaturated fatty acids. There are significant differences between the two. Also, long-chain fatty acids are mainly absorbed by lymphatic vessels in the intestine, while medium-chain fatty acids are mainly absorbed by intestinal mucosal cells. Therefore, we detected the kinetics of a sesame oil preparation of PAO orally administered to the monkeys and compared them with the kinetics of intravenous PAO.

Two groups of monkeys were selected, all of which were male. PAO was administered to a first group (C1001 and C1002) through iv injection by taking 1% DMSO as a vehicle at an actual dose of 0.118 mg/kg (nominal dose: 0.100 mg/kg) via single dosing. PAO was orally administered to a second group (C2001 and C2002) by taking sesame oil as a vehicle at an actual dose of 0.168 mg/kg (nominal dose: 0.200 mg/kg) via similarly single dosing. For each group, blood was collected at 0.083, 0.25, 0.5, 1, 2, 4, 8, 12, 24, and 48 hours after administration to detect the concentration of the compound in the blood (whole blood, not plasma).

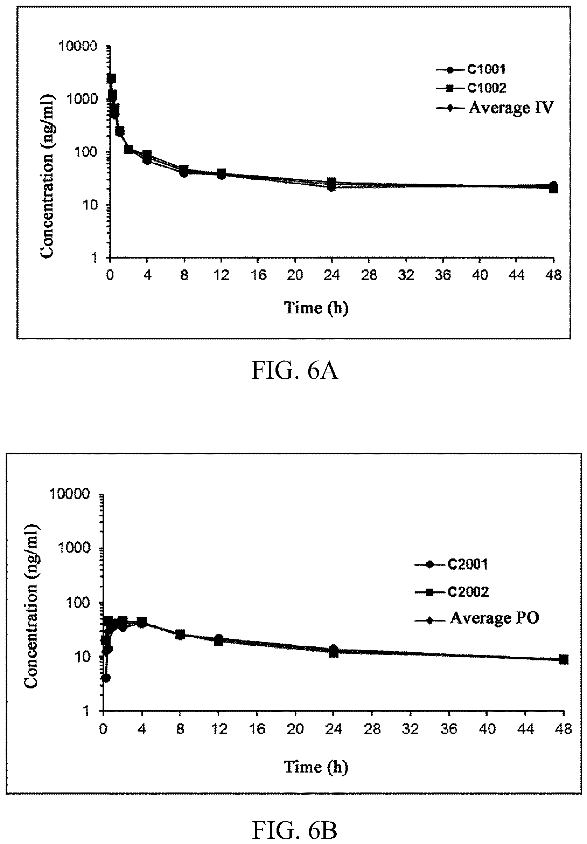

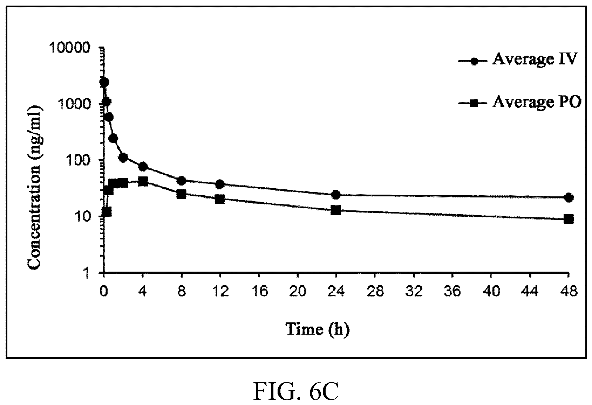

The results are shown in Table 53 and FIGs. 6A-6C:

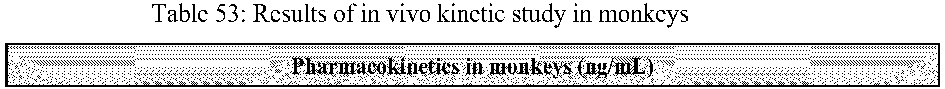

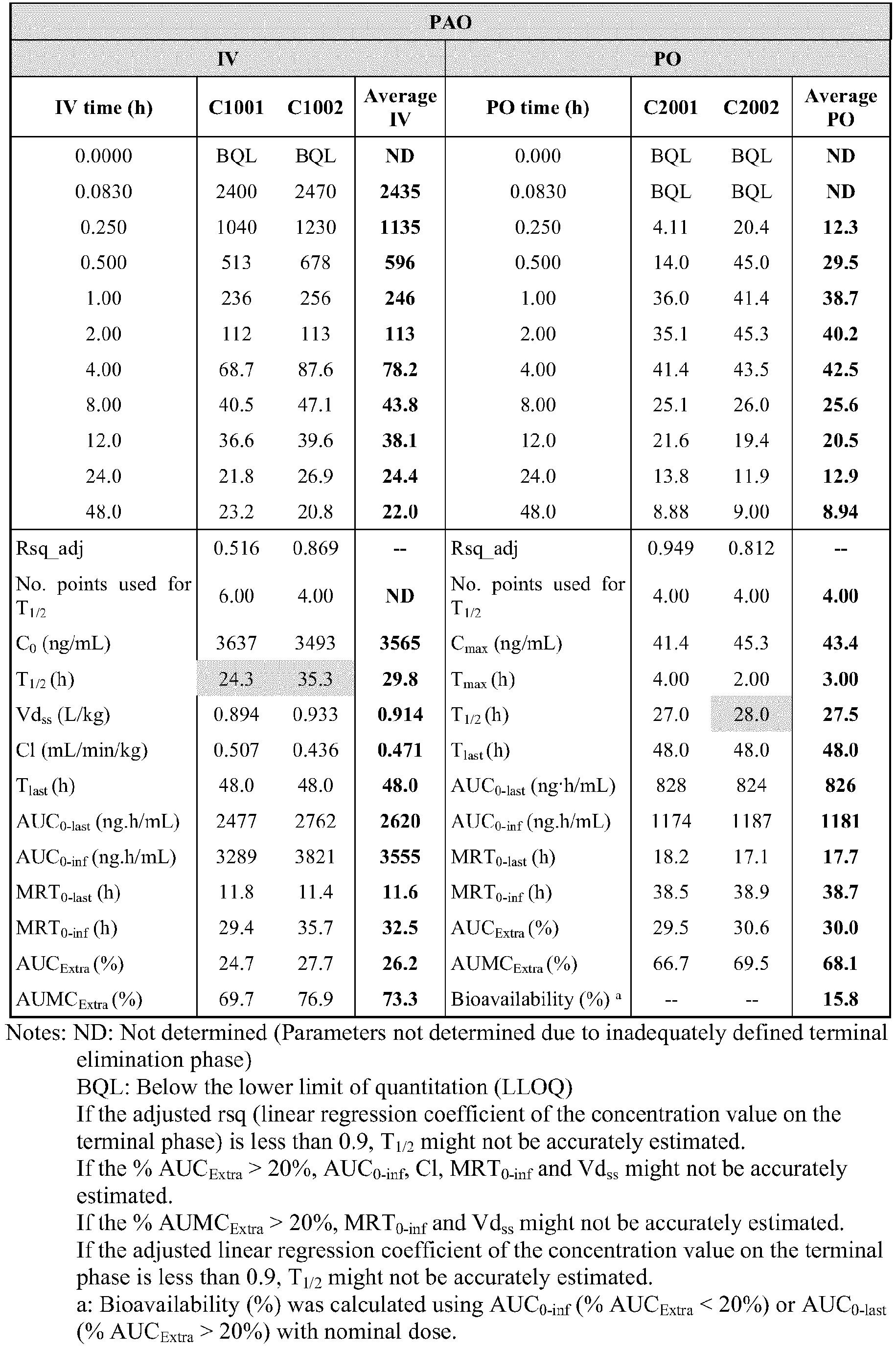

Analysis: After a single oral administration of the sesame oil preparation of PAO to the monkeys (at a dose of 0.168 mg/kg), PAO can also be absorbed into the blood, and the blood concentration similarly reached the maximum within 4 hours. The half life of PAO in the blood was about 27.5 hours. The average exposure of PAO in the blood (AUC₀₋ₗₐₛₜ = 826 ng·h/mL) was about 0.48 times as high as the average exposure of a single oral administration of the MCT preparation of PAO at 0.3 mg/kg (AUC₀₋ₗₐₛₜ = 1717 ng·h/mL) and about 0.25 times as high as the average exposure at 0.6 mg/kg (AUC₀₋ₗₐₛₜ = 3302 ng·h/mL). This indicated that the oral sesame oil preparation and MCT preparation of PAO were comparable in in vivo kinetics and bioavailability.

### 5.3 In vivo kinetic study of oral MCT preparation of PAO in beagles

Two groups of beagles were selected, all of which were male. PAO was administered to a first group (D1001 and D1002) through iv injection by taking 1% DMSO as a vehicle at an actual dose of 0.101 mg/kg (nominal dose: 0.100 mg/kg) via single dosing. PAO was orally administered to a second group (D2001 and D2002) by taking sesame oil as a vehicle at an actual dose of 0.169 mg/kg (nominal dose: 0.200 mg/kg) via similarly single dosing. For each group, blood was collected at 0.083, 0.25, 0.5, 1, 2, 4, 8, 12, 24, and 48 hours after administration to detect the concentration of the compound in the blood (whole blood, not plasma).

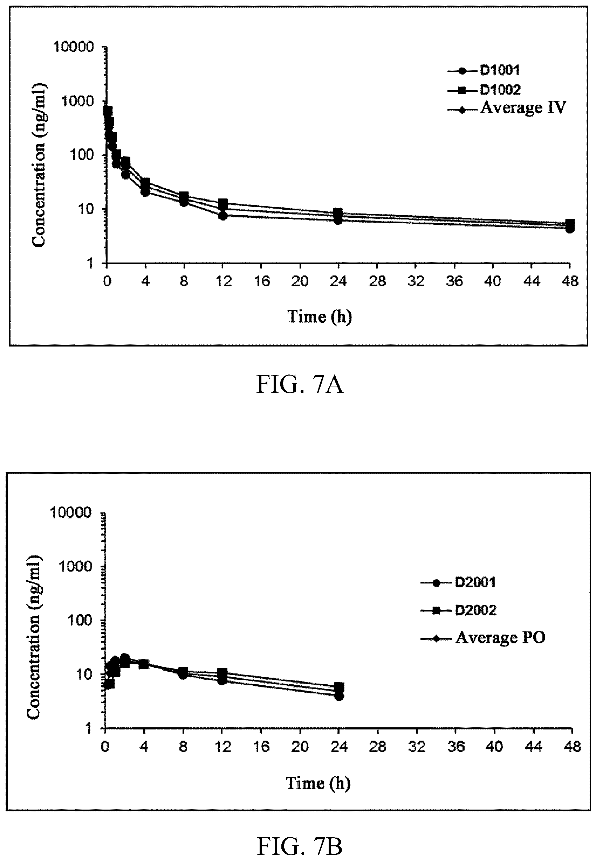

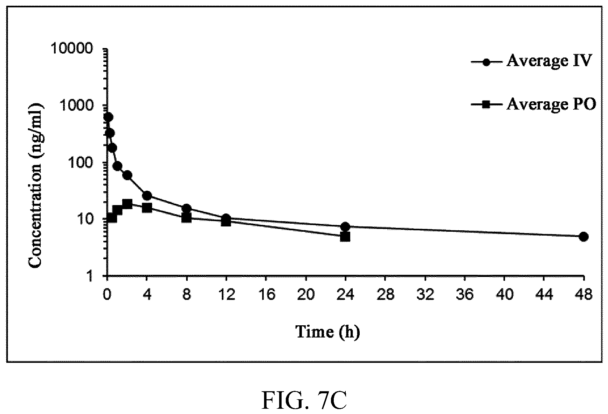

The results are shown in Table 54 and FIGs. 7A-7C:

**Table 54: Results of in vivo kinetic study in beagles**

| **Pharmacokinetics in beagles (ng/mL)** | | | | | | | |
|---|---|---|---|---|---|---|---|
| **PAO** | | | | | | | |
| **IV** | | | | **PO** | | | |
| **IV time (h)** | **D1001** | **D1002** | **Average IV** | **PO time (h)** | **D2001** | **D2002** | **Average PO** |
| 0.0000 | BQL | BQL | **ND** | 0.000 | BQL | BQL | **ND** |
| 0.0830 | 603 | 657 | **630** | 0.0830 | BQL | BQL | **ND** |
| 0.250 | 238 | 411 | **325** | 0.250 | 6.36 | BQL | **ND** |
| 0.500 | 146 | 219 | **183** | 0.500 | 14.5 | 6.72 | **10.6** |
| 1.00 | 68.4 | 102 | **85.2** | 1.00 | 18.1 | 10.9 | **14.5** |
| 2.00 | 43.2 | 75.0 | **59.1** | 2.00 | 20.6 | 16.7 | **18.7** |
| 4.00 | 20.6 | 31.5 | **26.1** | 4.00 | 16.1 | 15.5 | **15.8** |
| 8.00 | 13.5 | 17.4 | **15.5** | 8.00 | 9.81 | 11.3 | **10.6** |
| 12.0 | 7.65 | 12.9 | **10.3** | 12.0 | 7.62 | 10.6 | **9.11** |
| 24.0 | 6.27 | 8.49 | **7.38** | 24.0 | 4.02 | 5.88 | **4.95** |
| 48.0 | 4.41 | 5.46 | **4.94** | 48.0 | BQL | BQL | **ND** |
| Rsq_adj | 0.998 | 0.940 | -- | Rsq_adj | 0.997 | 0.967 | -- |
| No. points used for T_{1/2} | 3.00 | 3.00 | **3.00** | No. points used for T_{1/2} | 3,00 | 4.00 | **ND** |
| C₀ (ng/mL) | 957 | 830 | **893** | Cₘₐₓ (ng/mL) | 20.6 | 16.7 | **18.7** |
| T_{1/2} (h) | 45.6 | 30.0 | **37.8** | Tₘₐₓ (h) | 2.00 | 2.00 | **2.00** |
| Vdₛₛ (L/kg) | 4.42 | 2.27 | **3.35** | T_{1/2} (h) | 12.6 | 15.0 | **13.8** |
| Cl (mL/min/kg) | 1.75 | 1.42 | **1.59** | Tₗₐₛₜ (h) | 24.0 | 24.0 | **24.0** |
| Tₗₐₛₜ (h) | 48.0 | 48.0 | **48.0** | AUC₀₋ₗₐₛₜ (ng·h/mL) | 220 | 245 | **233** |
| AUC₀₋ₗₐₛₜ (ng.h/mL) | 662 | 936 | **799** | AUC_{0-inf} (ng.h/mL) | 293 | 372 | **333** |
| AUC_{0-inf} (ng.h/mL) | 952 | 1173 | **1062** | MRT₀₋ₗₐₛₜ (h) | 8.89 | 10.4 | **9.64** |
| MRT₀₋ₗₐₛₜ (h) | 10.7 | 10.3 | **10.5** | MRT_{0-inf} (h) | 17.1 | 22.4 | **19.8** |
| MRT_{0-inf} (h) | 42.1 | 26.6 | **34.4** | AUC_{Extra} (%) | 24.8 | 34.1 | **29.5** |
| AUC_{Extra} (%) | 30.5 | 20.2 | **25.3** | AUMC_{Extra} (%) | 61.0 | 69.5 | **65.3** |
| AUMC_{Extra} (%) | 82.3 | 69.1 | **75.7** | Bioavailability (%)^{a} | -- | -- | **14.6** |

| | | | | | | | |
|---|---|---|---|---|---|---|---|
| Notes: ND: Not determined (Parameters not determined due to inadequately defined terminal elimination phase) BQL: Below the lower limit of quantitation (LLOQ) If the adjusted rsq (linear regression coefficient of the concentration value on the terminal phase) is less than 0.9, T_{1/2} might not be accurately estimated. If the % AUC_{Extra} > 20%, AUC_{0-inf}, Cl, MRT_{0-inf} and Vdₛₛ might not be accurately estimated. If the % AUMC_{Extra} > 20%, MRT_{0_inf} and Vdₛₛ might not be accurately estimated. If the adjusted linear regression coefficient of the concentration value on the terminal phase is less than 0.9, T_{1/2} might not be accurately estimated. a: Bioavailability (%) was calculated using AUC_{0-inf} (% AUC_{Extra} < 20%) or AUC₀₋ₗₐₛₜ (% AUC_{Extra} > 20%) with nominal dose. | | | | | | | |

Analysis: After a single oral administration of the sesame oil preparation of PAO to the beagles, the blood concentration also reached the maximum within 4 hours, and the bioavailability was similar, being about 15%. However, the exposure of PAO in the blood of the beagles was about one fourth of the exposure in the blood of the monkeys. This indicated that the lipid preparation of PAO has similar bioavailability in different animal species, but has relatively large difference in exposure.

### 5.4 In vivo kinetic study of oral DMSO preparation and MCT preparation of PAO in mice

In order to compare the difference between the oral DMSO preparation and the oral MCT preparation, in vivo kinetic study in mice was carried out. Male mice were divided into two groups, 3 mice per group. PAO was orally administered to one group (M01, M02 and M03) by taking a 1% DMSO aqueous solution as a vehicle at an actual dose of 0.0913 mg/kg (nominal dose: 0.100 mg/kg). The MCT preparation of PAO was administered to the other group (N01, N02 and N03) at an actual dose of 0.107 mg/kg (nominal dose: 0.100 mg/kg). Blood was collected at 0.083, 0.25, 0.5, 1, 2, 4, 6, 8 and 24 hours after administration to detect the concentration of the compound in the blood (whole blood, not plasma).

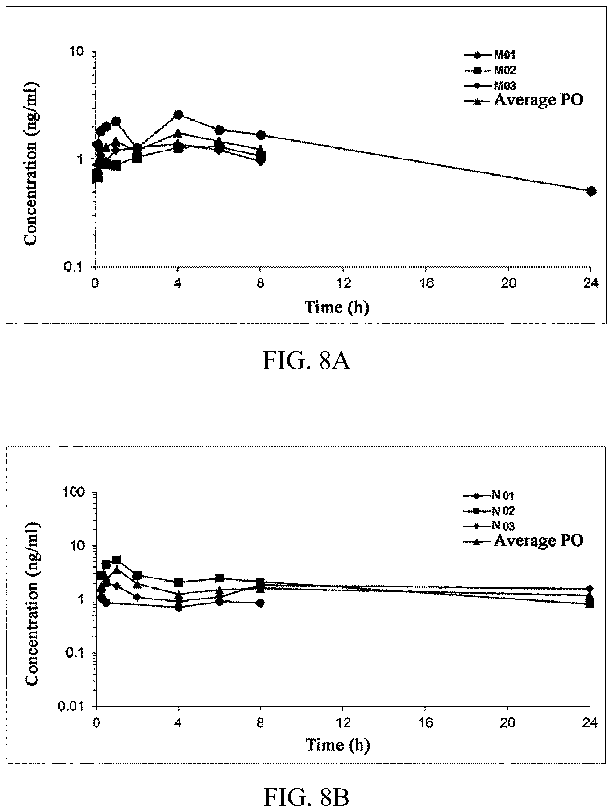

The results are shown in Tables 55-56 and FIGs. 8A-8B:

**Table 55: Results of in vivo kinetic study of oral 1% DMSO preparation of PAO (dose: 0.0913 mg/kg) in mice**

| **Pharmacokinetics of PAO in mice (ng/mL)** | | | | | | | |
|---|---|---|---|---|---|---|---|
| **PAO** | | | | | | | |
| **PO** | | | | | | | |
| **PO time (h)** | **M01** | **M02** | **M03** | **Average PO** | | **SD** | **CV (%)** |
| 0.0830 | 1.39 | 0.678 | 0.783 | **0.950** | ± | **0.384** | **40.4** |
| 0.250 | 1.82 | 0.916 | 1.08 | **1.27** | ± | **0.482** | **37.9** |
| 0.500 | 2.02 | 0.899 | 0.951 | **1.29** | ± | **0.633** | **49.0** |
| 1.00 | 2.27 | 0.874 | 1.22 | **1.45** | ± | **0.727** | **50.0** |
| 2.00 | 1.27 | 1.04 | 1.29 | **1.20** | ± | **0.139** | **11.6** |
| 4.00 | 2.59 | 1.29 | 1.38 | **1.75** | ± | **0.726** | **41.4** |
| 6.00 | 1.89 | 1.30 | 1.21 | **1.47** | ± | **0.369** | **25.2** |
| 8.00 | 1.69 | 1.07 | 0.954 | **1.24** | ± | **0.396** | **32.0** |
| 24.0 | 0.509 | BQL | BQL | **ND** | ± | **ND** | **ND** |

| **PK parameters** | **M01** | **M02** | **M03** | **Average PO** | | **SD** | **CV (%)** |
|---|---|---|---|---|---|---|---|
| Rsq_adj | 0.999 | ND | ND | -- | ± | -- | -- |
| No. points used for T_{1/2} | 3.00 | 0.00 | 0.00 | **ND** | ± | -- | -- |
| Cₘₐₓ (ng/mL) | 2.59 | 1.30 | 1.38 | **1.76** | ± | **0.723** | **41.1** |
| Tₘₐₓ (h) | 4.00 | 6.00 | 4.00 | **4.67** | ± | **1.15** | **24.7** |
| T_{1/2} (h) | 9.41 | ND | ND | **ND** | ± | **ND** | **ND** |
| Tₗₐₛₜ (h) | 24.0 | 8.00 | 8.00 | **ND** | ± | -- | -- |
| AUC₀₋ₗₐₛₜ (ng·h/mL) | 31.3 | 9.07 | 9.65 | **16.7** | ± | **12.7** | **75.9** |
| AUC_{0-inf} (ng.h/mL) | 38.2 | ND | ND | **ND** | ± | **ND** | **ND** |
| MRT₀₋ₗₐₛₜ (h) | 9.29 | 4.25 | 3.98 | **5.84** | ± | **2.99** | **51.2** |
| MRT_{0-inf} (h) | 14.4 | ND | ND | **ND** | ± | **ND** | **ND** |
| AUC_{Extra} (%) | 18.1 | ND | ND | **ND** | ± | **ND** | **ND** |
| AUMC_{Extra} (%) | 47.2 | ND | ND | **ND** | ± | **ND** | **ND** |

| | | | | | | | |
|---|---|---|---|---|---|---|---|
| Notes: ND: Not determined (Parameters not determined due to inadequately defined terminal elimination phase) BQL: Below the lower limit of quantitation (LLOQ) If the adjusted rsq (linear regression coefficient of the concentration value on the terminal phase) is less than 0.9, T_{1/2} might not be accurately estimated. If the % AUC_{Extra} > 20%, AUC_{0-inf}, Cl, MRT_{0-inf} and Vdₛₛ might not be accurately estimated. If the % AUMC_{Extra} > 20%, MRT_{0_inf} and Vdₛₛ might not be accurately estimated. If the adjusted linear regression coefficient of the concentration value on the terminal phase is less than 0.9, T_{1/2} might not be accurately estimated. | | | | | | | |

**Table 56: Results of in vivo kinetic study of oral MCT preparation of PAO (dose: 0.107 mg/kg) in mice**

| **Pharmacokinetics of PAO in mice (ng/mL)** | | | | | | | |
|---|---|---|---|---|---|---|---|
| **PAO** | | | | | | | |
| **PO** | | | | | | | |
| **PO time (h)** | **N01** | **N02** | **N03** | **Average PO** | | **SD** | **CV (%)** |
| 0.0830 | BQL | BQL | BQL | **ND** | ± | **ND** | **ND** |
| 0.250 | 1.06 | 2.78 | 1.42 | **1.75** | ± | **0.907** | **51.7** |
| 0.500 | 0.875 | 4.57 | 1.95 | **2.47** | ± | **1.90** | **77.1** |
| 1.00 | BQL | 5.47 | 1.80 | **3.64** | ± | **ND** | **ND** |
| 2.00 | BQL | 2.81 | 1.10 | **1.96** | ± | **ND** | **ND** |
| 4.00 | 0.714 | 2.07 | 0.926 | **1.24** | ± | **0.729** | **59.0** |
| 6.00 | 0.916 | 2.47 | 1.12 | **1.50** | ± | **0.844** | **56.2** |
| 8.00 | 0.857 | 2.12 | 1.85 | **1.61** | ± | **0.665** | **41.3** |
| 24.0 | BQL | 0.822 | 1.57 | **1.20** | ± | **ND** | **ND** |

| **PK parameters** | **N01** | **N02** | **N03** | **Average PO** | | **SD** | **CV (%)** |
|---|---|---|---|---|---|---|---|
| Rsq_adj | ND | 0.999 | ND | -- | ± | -- | -- |
| No. points used for T_{1/2} | 0.00 | 3.00 | 0.00 | **ND** | ± | -- | -- |
| Cₘₐₓ (ng/mL) | 1.06 | 5.47 | 1.95 | **2.83** | ± | **2.33** | **82.5** |
| Tₘₐₓ (h) | 0.250 | 1.00 | 0.500 | **0.583** | ± | **0.382** | **65.5** |
| Tᵣ₂ (h) | ND | 11.5 | ND | **ND** | ± | **ND** | **ND** |
| Tₗₐₛₜ (h) | 8.00 | 24.0 | 24.0 | **ND** | ± | -- | -- |
| AUC₀₋ₗₐₛₜ (ng·h/mL) | 6.54 | 43.6 | 37.2 | **29.1** | ± | **19.8** | **68.1** |
| AUC_{0-inf} (ng.h/mL) | ND | 57.3 | ND | **ND** | ± | **ND** | **ND** |
| MRT₀₋ₗₐₛₜ (h) | 4.09 | 9.18 | 12.6 | **8.64** | ± | **4.30** | **49.8** |
| MRT_{0_inf} (h) | ND | 16.6 | ND | **ND** | ± | **ND** | **ND** |
| AUC_{Extra} (%) | ND | 23.8 | ND | **ND** | ± | **ND** | **ND** |
| AUMC_{Extra} (%) | ND | 57.9 | ND | **ND** | ± | **ND** | **ND** |

| | | | | | | | |
|---|---|---|---|---|---|---|---|
| Notes: ND: Not determined (Parameters not determined due to inadequately defined terminal elimination phase) BQL: Below the lower limit of quantitation (LLOQ) If the adjusted rsq (linear regression coefficient of the concentration value on the terminal phase) is less than 0.9, T_{1/2} might not be accurately estimated. If the % AUC_{Extra} > 20%, AUC_{0-inf}, Cl, MRT_{0-inf} and Vdₛₛ might not be accurately estimated. If the % AUMC_{Extra} > 20%, MRT_{0_inf} and Vdₛₛ might not be accurately estimated. If the adjusted linear regression coefficient of the concentration value on the terminal phase is less than 0.9, T_{1/2} might not be accurately estimated. | | | | | | | |

The results showed that the bioavailability of PAO delivered by the MCT preparation was apparently higher than the bioavailability of PAO delivered by the cosolvent DMSO aqueous solution. In addition, Example 4.2 and Table 42 of the present invention showed that the PAO was released from its MCT preparation in a sustained manner in the simulated gastric juice. Therefore, the lipid preparation of PAO can not only realize the sustained release of PAO in the gastric juice so as to relieve irritation of PAO to the gastric mucosa, but also increase the bioavailability of PAO.

### 5.5 In vivo kinetic study of PAO in rats

In order to compare the differences between PAO preparations in administration route, dose and gender of administered subjects, in vivo kinetic study in rats was carried out. Rats were divided into four groups, 6 rats per group. Each group included 3 female rats and 3 male rats. PAO was intravenously administered to a first group at a nominal dose of 0.1 mg/kg. PAO was orally administered to a second group at a nominal dose of 0.1 mg/kg. PAO was orally administered to a third group at a nominal dose of 0.3 mg/kg. PAO was orally administered to a fourth group at a nominal dose of 0.9 mg/kg. The concentration of the compound in the blood (whole blood, not plasma) within 36 hours after administration was detected.

The results are shown in Table 57:

**Table 57: Average pharmacokinetic parameters of PAO in male and female**

| SD rats (n=6) | | | | | | | | |
|---|---|---|---|---|---|---|---|---|
| **Group** | **1** | | **2** | | **3** | | **4** | |
| **Administration route** | **IV** | | **PO** | | **PO** | | **PO** | |
| **Dose level (mg/kg)** | **0.1** | | **0.1** | | **0.3** | | **0.9** | |
| **PK parameters** | **Average** | **SD** | **Average** | **SD** | **Average** | **SD** | **Average** | **SD** |
| **C₀ or Cₘₐₓ (ng/mL)** | 1390 | 130 | 71.7 | 30.2 | 262 | 55.1 | 665 | 174 |
| **Tₘₐₓ (h)** | -- | -- | 4.67 | 1.03 | 5.33 | 2.07 | 7.00 | 3.03 |
| **T_{1/2} (h)** | 7.59 | 0.992 | 7.26 | 1.11 | 6.83 | 0.769 | 7.68 | 1.59 |
| **Vdₛₛ (L/kg)** | 0.130 | 0.011 4 | -- | -- | -- | -- | -- | -- |
| **CL (mL/min/kg)** | 0.343 | 0.031 2 | -- | -- | -- | -- | -- | -- |
| **AUC_{0.last} (h•ng/mL)** | 4790 | 432 | 739 | 288 | 2950 | 694 | 9120 | 2060 |
| **AUC_{0-inf} (h•ng/mL)** | 4890 | 436 | 767 | 294 | 3050 | 700 | 9710 | 1960 |
| **Bioavailability (%)** | -- | -- | 15.7 | -- | 20.8 | -- | 22.1 | -- |

| | | | | | | | | |
|---|---|---|---|---|---|---|---|---|
| a: Bioavailability (%) was calculated with average AUC_{0-inf} and nominal dose. "--" means not applicable | | | | | | | | |

**Table 58: Dose ratio of PAO in male and female SD rats after single oral administration of PAO**

| **Gender** | **Compared Doses (mg/kg)** | | | **Dose ratio** | **Cₘₐₓ value** | **Cₘₐₓ ratio** | **AUC₀₋ₗₐₛₜ value** | **AUC₀₋ last ratio** |
|---|---|---|---|---|---|---|---|---|
| Male | 0.300 | over | 0.100 | 3.00 | 230/59.6 | 3.86 | 2560/666 | 3.84 |
| | 0.900 | over | 0.300 | 3.00 | 580/230 | 2.52 | 8270/2560 | 3.23 |
| | 0.900 | over | 0.100 | 9.00 | 580/59.6 | 9.73 | 8270/666 | 12.4 |
| Female | 0.300 | over | 0.100 | 3.00 | 294/83.8 | 3.51 | 3330/812 | 4.10 |
| | 0.900 | over | 0.300 | 3.00 | 749/294 | 2.55 | 9970/3330 | 2.99 |
| | 0.900 | over | 0.100 | 9.00 | 749/83.8 | 8.94 | 9970/812 | 12.3 |

**Table 59: Gender comparison of PAO on systemic exposure in male and female SD rats after single intravenous or oral administration of PAO**

| **Administration route** | **Dose level (mg/kg)** | **C₀ or Cₘₐₓ (female and male)** | **C₀ or Cₘₐₓ ratio (female/male)** | **AUC₀₋ₗₐₛₜ value (female and male)** | **AUC₀₋ₗₐₛₜ ratio (female/male)** |
|---|---|---|---|---|---|
| IV | 0.100 | 1420/1350 | 1.05 | 4630/4940 | 0.937 |
| PO | 0.100 | 83.8/59.6 | 1.41 | 812/666 | 1.22 |
| PO | 0.300 | 294/230 | 1.28 | 3330/2560 | 1.30 |
| PO | 0.900 | 749/580 | 1.29 | 9970/8270 | 1.21 |

### 5.6 In vivo kinetic study of PAO in rats

In order to study the influences of ethanol on the oral MCT preparation of PAO, in vivo kinetic study in rats was carried out. Rats were divided into two groups, 2 rats per group, one male and one female in each group. MCT preparations of PAO (PAO in MCT solution, having a concentration of 1.5 mg/g) were orally administered to one group (R01 and R02) by using a vehicle containing no ethanol at a dose of 0.1 mpk. MCT preparations of PAO (containing 1.05% (v/v) of ethanol) were orally administered to the other group (R01 and R02) at a dose of 0.2 mpk. Blood was collected at 0.25, 0.5, 1, 2, 4, 6, 8 and 24 hours after administration to detect the concentration of the compound in the blood.

The results are shown in Table 60:

**Table 60: Influences of ethanol on the PK of MCT preparations of PAO orally administered to SD rats**

| Vehicle (containing no ethanol) | | | DMPK formulation (containing 1.05% (v/v) of ethanol) | |
|---|---|---|---|---|
| At a dose of 0.1 mpk | | | At a dose of 0.2 mpk | |
| | PO1 (male) | PO2 (female) | PO1 (male) | PO2 (female) |
| PO time (h) | R01 | R02 | R01 | R02 |
| 0.250 | 3.73 | 4.35 | 14.4 | 20.0 |
| 0.500 | 8.89 | 7.93 | 30.2 | 49.2 |
| 1.00 | 11.7 | 12.0 | 47.1 | 64.8 |
| 2.00 | 17.4 | 11.2 | 61.4 | 117 |
| 4.00 | 17.8 | 14.0 | 98.3 | 210 |
| 6.00 | 14.4 | 13.7 | 121 | 193 |
| 8.00 | 10.6 | 11.9 | 96.2 | 140 |
| 24.0 | 6.58 | 3.69 | 11.0 | 15.4 |
| PK parameters | R01 | R02 | R01 | R02 |
| Rsq_adj | 0.829 | 1.00 | 1.00 | 1.00 |
| No. points used for T_{1/2} | 3.00 | 3.00 | 3.00 | 3.00 |
| Cₘₐₓ (ng/mL) | 17.8 | 14.0 | 121 | 210 |
| Tₘₐₓ (h) | 4.00 | 4.00 | 6.00 | 4.00 |
| T_{1/2} (h) | 18.1 | 9.50 | 5.17 | 4.97 |
| Tₗₐₛₜ (h) | 24.0 | 24.0 | 24.0 | 24.0 |
| AUC₀₋ₗₐₛₜ (ng·h/mL) | 249 | 209 | 1305 | 2094 |
| AUC_{0-inf} (ng.h/mL) | 421 | 260 | 1387 | 2204 |
| MRT₀₋ₗₐₛₜ (h) | 10.2 | 9.75 | 8.90 | 8.38 |
| MRT_{0-inf} (h) | 26.5 | 15.2 | 10.2 | 9.52 |
| AUC_{Extra} (%) | 40.9 | 19.5 | 5.91 | 5.01 |
| AUMC_{Extra} (%) | 77.3 | 48.3 | 18.2 | 16.4 |

**Table 61: Summary of influences of ethanol on the PK of MCT preparations of PAO orally administered to SD rats**

| Preparation | Dose | AUC₀₋ₗₐₛₜ | Average | Compared with the preparation containing no ethanol at the same dose |
|---|---|---|---|---|
| Containing no ethanol | 0.1 | 249 209 | 229 | |
| Containing ethanol | 0.2 | 1305 | 1700 | 3.71 |
| | | 2094 | | |

It can be seen that adding ethanol to the MCT preparation of PAO can increase the exposure of oral PAO in the blood or increase the bioavailability of oral PAO. When the concentration of the ethanol was 1.05% (v/v), the bioavailability can be increased by 2-3 times.

### Example 6: Toxicity study of different PAO preparations in animals

In order to compare the toxicity of an MCT preparation of PAO and a 0.1% DMSO aqueous solution of PAO in animals, 20 male ICR mice and 20 female ICR mice were selected, all of which were 10 weeks old. The male and female mices were equally divided into 4 groups to which the MCT preparation of PAO and the 0.1% DMSO aqueous solution (v/v) of PAO were intragastrically administered at 1.5 or 0.75 mg/kg/day respectively for 46 days (Conditions for grouping and dosing of mice are shown in Table 62). The mice were weighed every day, and dead mice were documented.

**Table 62: Grouping and dosing of mice**

| Preparation | Dose | Number of female mice | Number of male mice |
|---|---|---|---|
| MCT preparation | 1.5 mg/kg/day | 5 | 5 |
| | 0.75 mg/kg/day | 5 | 5 |
| 0.1% DMSO aqueous solution | 1.5 mg/kg/day | 5 | 5 |
| | 0.75 mg/kg/day | 5 | 5 |

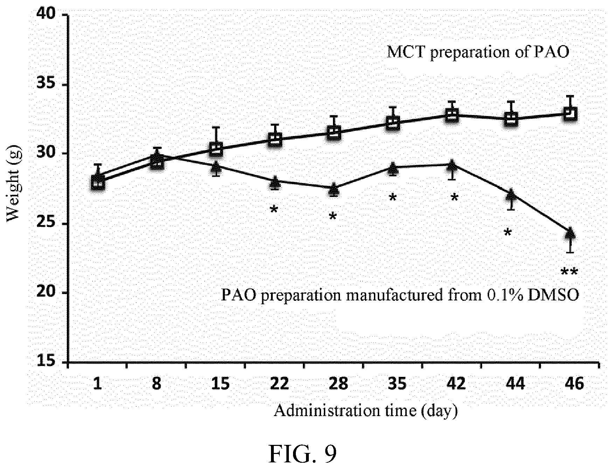

After consecutive administration for 46 days, all the female mice survived. The average weights of the two groups of female mice to which the MCT preparation and the 0.1% DMSO aqueous solution of PAO were orally administered at 0.75 mg/kg/day increased slowly, and there was almost no difference between the two groups when the administration was completed (30.6 g and 30.2 g). The average weights of the female mice to which the MCT preparation of PAO was orally administered at 1.5 mg/kg/day slowly increased to 32.9 g. However, the average weights of the mice to which the 0.1% DMSO aqueous solution of PAO was orally administered decreased significantly after the second week, and finally dropped to 24.4 g (FIG. 9). As for the male mice, every mouse to which the MCT preparation of PAO was orally administered at 0.75 mg/kg/day survived, and the weight of each mouse increased slowly. However, there was no obvious regularity for the weight changes of the mice in the other three groups. The specific results were as follows: one of the 5 mice to which the MCT preparation of PAO was orally administered at 1.5 mg/kg/day died in the second week of administration; one of the 5 mice to which the 0.1% DMSO aqueous solution of PAO was orally administered at 0.75 mg/kg/day died in the second week of administration; and two of the 5 mice to which the 0.1% DMSO aqueous solution of PAO was orally administered at 1.5 mg/kg/day died in the second and sixth week of administration, respectively.

These results showed that although the bioavailability of PAO delivered by the MCT preparation was apparently higher than the bioavailability of PAO delivered by the aqueous solution of cosolvent DMSO, the in vivo toxicity of the MCT preparation of PAO was significantly lower.

## Claims

1. A pharmaceutical composition, comprising a micromolecule PI4KIIIα inhibitor and a pharmaceutically acceptable carrier, wherein the pharmaceutically acceptable carrier comprises a lipid.

2. The pharmaceutical composition according to claim 1, wherein the micromolecule PI4KIIIα inhibitor is PAO and a derivative of PAO.

3. The pharmaceutical composition according to claim 1, wherein the micromolecule PI4KIIIα inhibitor has a structure of formula (I) or a pharmaceutically acceptable salt thereof, 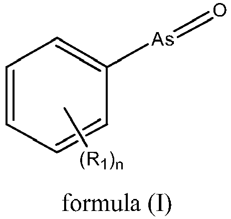 wherein R₁ is each independently selected from H, halogen, nitro, cyano, hydroxyl, amino, carbamoyl, C₁₋₆ alkyl, C₁₋₆ alkoxy, C₁₋₆ haloalkyl, -As(O), -NH-(C₁₋₆ alkyl), N,N-(C₁₋₆ alkyl)₂, -NH-C(O)-R₂, -NH-S(O)₂-R₃, -C(O)OR₄ or heterocyclyl, wherein n is an integer of 0-5, R₂ and R₃ are each independently selected from H, amino, C₁₋₆ alkyl, C₁₋₆ alkoxy, C₁₋₆ haloalkyl, -NH-(C₁₋₆ alkyl), N,N-(C₁₋₆ alkyl)₂,-C(O)OR₄, C₃₋₆ cycloalkyl, 6-12 membered aryl or 3-6 membered heterocyclyl, which are optionally substituted by halogen, nitro, cyano, hydroxyl, amino, carbamoyl, aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, and R₄ is C₁₋₆ alkyl.

4. The pharmaceutical composition according to claim 3, wherein R₁ is each independently selected from H, halogen, nitro, cyano, hydroxyl, amino, carbamoyl, C₁₋₆ alkyl, C₁₋₆ alkoxy, C₁₋₆ haloalkyl, -As(O), -NH-(C₁₋₆ alkyl), N,N-(C₁₋₆ alkyl)₂ or -C(O)OR₄, wherein n is an integer of 0-2, and R₄ is C₁₋₆ alkyl.

5. The pharmaceutical composition according to claim 3, wherein R₁ is each independently selected from H, halogen, nitro, cyano, hydroxyl, amino, C₁₋₆ alkyl, C₁₋₆ alkoxy, C₁₋₆ haloalkyl or -As(O), wherein n is an integer of 0-2.

6. The pharmaceutical composition according to claim 3, wherein R₁ is each independently selected from H, halogen, amino or C₁₋₆ alkoxy, wherein n is 1.

7. The pharmaceutical composition according to claim 6, wherein R₁ is located at an ortho position or a para position of the -As(O) group.

8. The pharmaceutical composition according to claim 3, wherein R₁ is H.

9. The pharmaceutical composition according to claim 1, wherein the micromolecule PI4KIIIα inhibitor is at an amount of 0.01-20 mg/g, 0.05-20 mg/g, 0.1-20 mg/g, 0.2-20 mg/g, 0.5-20 mg/g, 0.8-20 mg/g, 1-20 mg/g, 1-18 mg/g, 1-16 mg/g, 1-14 mg/g, 1-12 mg/g, 1-10 mg/g, 2-10 mg/g, 2-8 mg/g, 2-6 mg/g, 3-6 mg/g, 0.2-15 mg/g, 0.2-12 mg/g, 0.2-10 mg/g, 0.2-8 mg/g, 0.2-6 mg/g, 0.2-4 mg/g, 0.2-2 mg/g, 0.2-1 mg/g or 0.2-0.8 mg/g in the pharmaceutical composition.

10. The pharmaceutical composition according to claim 1, wherein the pharmaceutically acceptable carrier comprises at least about 50% (w/w), at least about 60% (w/w), at least about 70% (w/w), at least about 80% (w/w), at least about 85% (w/w), at least about 90% (w/w), at least about 95% (w/w), at least about 97% (w/w), at least about 98% (w/w), at least about 99% (w/w) or 100% (w/w) of the lipid.

11. The pharmaceutical composition according to any one of the preceding claims, wherein the lipid comprises a lipid with a melting point of -20-80°C, -20-10°C or -20-0°C.

12. The pharmaceutical composition according to any one of the preceding claims, wherein the lipid has a degree of unsaturation of 0-5, 0-4, 0-3, 0-2, 0-1 or 0.

13. The pharmaceutical composition according to any one of the preceding claims, wherein the lipid comprises a lipid which has a fatty acid carbon chain at a length in a range of 4-24, 4-22, 4-20, 6-20, 6-16, 6-14, 6-13, 6-12, 8-13, 8-12 or 8-10 carbon atoms.

14. The pharmaceutical composition according to any one of the preceding claims, wherein the lipid comprises a lipid which has a fatty acid chain at a length of 8 and 10, and optionally further comprises a lipid which has the fatty acid carbon chain at a length of 12-22.

15. The pharmaceutical composition according to any one of the preceding claims, wherein the fatty acid chain in the lipid is a long-chain fatty acid, a medium-chain fatty acid or a short-chain fatty acid.

16. The composition according to claim 1, wherein the lipid is vegetable oil.

17. The pharmaceutical composition according to claim 16, wherein the vegetable oil is olive oil, tea oil, rapeseed oil, peanut oil, soybean oil, corn oil, safflower oil, groundnut oil, sunflower seed oil, canola oil, walnut oil, almond oil, avocado oil, castor oil, coconut oil, cottonseed oil, rice bran oil, sesame oil, refined palm oil or a mixture thereof.

18. The pharmaceutical composition according to any one of the preceding claims, wherein the lipid is a fatty acid, a fatty acid ester, a fatty alcohol, a lipoid, a paraffin or a mixture thereof.

19. The pharmaceutical composition according to claim 18, wherein the lipoid is a phospholipid, a sucrose ester, a steroid, a fat-soluble vitamin or a mixture thereof.

20. The pharmaceutical composition according to claim 18, wherein the fatty acid ester is a glyceride, an ethylene glycol ester, a propylene glycol ester or a mixture thereof.

21. The pharmaceutical composition according to claim 18, wherein the fatty acid ester is a monoester, a diester, a triester or a mixture thereof.

22. The pharmaceutical composition according to claim 18, wherein the fatty acid ester comprises glycerides of octanoic acid and/or decanoic acid.

23. The pharmaceutical composition according to claim 18, wherein the fatty acid ester comprises a medium-chain triglyceride.

24. The pharmaceutical composition according to any one of the preceding claims, wherein the pharmaceutically acceptable carrier further comprises an antioxidant.

25. The pharmaceutical composition according to claim 24, wherein the antioxidant is at an amount of 0.001 %-5% (wt), 0.005%-5% (wt), 0.01%-5% (wt), 0.05%-5% (wt), 0.1%-5% (wt), 0.1%-3% (wt), 0.1%-2% (wt), 0.1%-1% (wt), 0.1%-0.8% (wt), 0.1%-0.5% (wt), 0.1%-0.3% (wt), 0.3%-2% (wt), 0.5%-2% (wt), 0.8%-2% (wt) or 1%-2% (wt) based on the weight of the pharmaceutical composition.

26. The pharmaceutical composition according to claim 24, wherein the antioxidant is sulfite, bisulfite, pyrosulfite, dithiocarbamate, ascorbic acid, ascorbyl palmitate, hydrocoumarin, vitamin E, ethanolamine, propyl gallate, butylated hydroxyanisole (BHA), butylated hydroxytoluene (BHT), nordihydroguaiaretic acid or glutathione.

27. The pharmaceutical composition according to any one of the preceding claims, wherein the pharmaceutically acceptable carrier further comprises a viscosity modifier, a pH regulator or a flavoring agent.

28. The pharmaceutical composition according to any one of the preceding claims, wherein the pharmaceutically acceptable carrier further comprises ethanol.

29. The pharmaceutical composition according to claim 28, wherein the ethanol is at an amount of 10%-0.1% (v/v).

30. The pharmaceutical composition according to claim 28, wherein the ethanol is at an amount of 8%-0.1 % (v/v), 7%-0.1 % (v/v), 6%-0.1 % (v/v), 5%-0.1% (v/v), 4%-0.1 % (v/v), 3%-0.1% (v/v), 2%-0.1 % (v/v), 1.5%-0.1 % (v/v), 1.2%-0.1% (v/v), 8%-0.3%(v/v), 8%-0.5%(v/v), 8%-0.7%(v/v), 8%-0.9% (v/v), 8%-1% (v/v), 6%-0.3% (v/v), 5%-0.5% (v/v), 4%-0.8% (v/v), 3%-0.9% (v/v) or 2%-1% (v/v).

31. The pharmaceutical composition according to any one of the preceding claims, wherein the pharmaceutical composition is used for oral, subcutaneous, intramuscular or intravenous administration.

32. The pharmaceutical composition according to any one of the preceding claims, wherein the pharmaceutical composition is tablets, capsules, suspensions, emulsions, solutions, semisolid preparations, patches or microneedles.

33. The pharmaceutical composition according to claim 1, wherein the micromolecule PI4KIIIα inhibitor is phenylarsine oxide, the phenylarsine oxide is at an amount of 0.25-20 mg/g in the pharmaceutical composition, and the pharmaceutically acceptable carrier is consisting of a medium-chain triglyceride, consisting of a medium-chain triglyceride and a long-chain triglyceride, or consisting of a medium-chain triglyceride and ethanol.

34. A method for preparing the pharmaceutical composition according to any one of claims 1-33, wherein the method comprises: mixing the micromolecule PI4KIIIα inhibitor and the pharmaceutically acceptable carrier to obtain a mixture.

35. The method according to claim 34, wherein the method comprises: mixing the micromolecule PI4KIIIα inhibitor and the pharmaceutically acceptable carrier through a mechanical force.

36. The method according to claim 35, wherein the mechanical force is stirring, dispersing, shaking or ultrasonic treatment.

37. The method according to claim 34, wherein the method comprises: mixing the micromolecule PI4KIIIα inhibitor and the pharmaceutically acceptable carrier after melting the pharmaceutically acceptable carrier by heating.

38. The method according to claim 37, further comprising: filtering the mixture.

39. A method for treating a PI4KIIIα-related disease in a subject, wherein the method comprises administrating the pharmaceutical composition according to any one of claims 1-33 to a subject in need thereof.

40. The method according to claim 39, wherein the PI4KIIIα-related disease is Alzheimer's disease.

41. The method according to claim 39, wherein the subject is an animal such as a pig, a dog, a monkey, a cat, a mouse, or a rat, or a human.

42. Use of the pharmaceutical composition according to any one of claims 1-33 in the manufacture of a medicament for treating a PI4KIIIα-related disease in a subject.

43. The pharmaceutical composition according to any one of claims 1-33 for use in treating a PI4KIIIα-related disease in a subject.
